# EUROPEAN PATENT SPECIFICATION

(11) **EP 1 487 966 B1**
(45) Date of publication and mention of the grant of the patent: **13.12.2006**
(21) Application number: 03716803.6
(22) Date of filing: 24.03.2003
(51) Int. Cl.: C12N 1/20

(54) **SECRETION OF PROTEINS WITH MULTIPLE DISULFIDE BONDS IN BACTERIA AND USES THEREOF**
SEKRETION VON PROTEINEN MIT MEHREREN DISULFIDBINDUNGEN IN BAKTERIEN UND VERWENDUNGEN DAVON
SECRETION DE PROTEINES A PLUSIEURS LIAISONS DISULFURE CHEZ DES BACTERIES ET LEUR UTILISATION

(30) Priority: 23.03.2002 US 367130 P
(43) Date of publication of application: 22.12.2004
(73) Proprietor: RESEARCH DEVELOPMENT FOUNDATION, Carson City, Nevada 89703 (US)
(72) Inventor: GEORGIOU, George, Austin, TX 78731 (US); MASIP, Lluis, Austin, TX 78731-3107-94 (US); DELISA, Matthew, Ithaca, NY 4850-2438 (US)
(74) Representative: McQueen, Andrew Peter
(86) International application number: PCT/US2003/008969
(87) International publication number: WO 2003/083056

(56) References cited:
- US-A- 6 008 023
- US-B1- 6 277 375
- SCHAERLAEKENS KRISTIEN ET AL: "Twin-arginine translocation pathway in Streptomyces lividans" JOURNAL OF BACTERIOLOGY, vol. 183, no. 23, December 2001 (2001-12), pages 6727-6732, XP002346951 ISSN: 0021-9193
- THOMAS J D ET AL: "Export of active green fluorescent protein to the periplasm by the twin-arginine translocase (Tat) pathway in Escherichia coli" MOLECULAR MICROBIOLOGY, BLACKWELL SCIENTIFIC, OXFORD, GB, vol. 39, no. 1, January 2001 (2001-01), pages 47-53, XP002330124 ISSN: 0950-382X
- BERKS B C ET AL: "THE TAT PROTEIN EXPORT PATHWAY" MOLECULAR MICROBIOLOGY, BLACKWELL SCIENTIFIC, OXFORD, GB, vol. 35, no. 2, January 2000 (2000-01), pages 260-274, XP008012416 ISSN: 0950-382X
- WU L-F ET AL: "BACTERIAL TWIN-ARGININE SIGNAL PEPTIDE-DEPENDENT PROTEIN TRANSLOCATION PATHWAY: EVOLUTION AND MECHANISM" JOURNAL OF MOLECULAR MICROBIOLOGY AND BIOTECHNOLOGY, HORIZON SCIENTIFIC PRESS, WYMONDHAM,, GB, vol. 2, no. 2, 2000, pages 179-189, XP008012409 ISSN: 1464-1801
- STANLEY ET AL.: 'Behavior of topological marker proteins targeted to the tat protein transport pathway' MOLECULAR MICROBIOLOGY vol. 43, no. 4, February 2002, pages 1005 - 1021, XP002971646
- TJALSMA ET AL.: 'Signal peptide-dependent protein transport in bacillus subtilis: a genome-based survey of the secretome' MICROBIOLOGY AND MOLECULAR BIOLOGY REVIEWS vol. 64, no. 3, September 2000, pages 515 - 547, XP002227136
- DELISA ET AL.: 'Genetic analysis of the twin arginine translocator secretion pathway in bacteria' THE JOURNAL OF BIOLOGICAL CHEMISTRY vol. 277, no. 33, 16 August 2002, pages 29825 - 29831, XP002971647
- BUCHANAN ET AL.: 'A genetic screen for suppressors of escherichia coli tat signal peptide mutations establishes a critical role for the second arginine within the twin-arginine motif' ARCH. MICROBIOL. vol. 177, 2001, pages 107 - 112, XP002971648

## Description

### 1. Field of the Invention

The present invention relates generally to the field of protein secretion. More specifically, the present invention relates to secretion of recombinant proteins with multiple disulfide bonds through the Twin-Arginine Translocation pathway in bacteria.

### 2. Description of Related Art

In bacteria, the bulk of protein secretion across the cytoplasmic membrane occurs via the *sec* pathway (Stuart and Neupert, 2000). Proteins transverse the membrane though the *"se*c translocon," a complex of three integral membrane proteins (SecY, SecE and SecG) which form a narrow diameter pore that serves as a conduit for the transport of proteins across the cytoplasmic membrane. Secreted polypeptides are largely unfolded, possibly resembling a random coil, and effectively thread their way through the pore in a process that requires the consumption of ATP. Notably, secretion of proteins in eukaryotes is fundamentally analogous, also involving the threading of an unfolded polypeptide though a narrow membrane pore, concomitant with ATP hydrolysis (Schatz and Dobberstein, 1996).

The *sec* pathway of protein export (and the analogous pathway in eukaryotes) has been studied over the last 25 years. Since no other secretion pathway was known until recently, secretory protein production for biotechnology purposes has relied exclusively on the *sec* pathway. The significance of the *sec* pathway for biotechnology purposes is highlighted by the vast literature relating to various leader peptides useful for protein expression and by the variety of strategies for improving the flux of secreted protein. However protein secretion via the *sec* pathway is subject to several intrinsic limitations. For example, a number of heterologous polypeptides are incompatible with transport via the *sec* pathway and thus cannot be expressed in secreted form. Incompatible polypeptides insert into the SecYEG pore where they become stuck and, as a result, cannot be completely translocated across the membrane. In turn, the secretory apparatus becomes jammed and unavailable for the trafficking of native proteins, a phenomenon that causes cessation of growth and, ultimately, cell lysis. Cell toxicity effects associated with the expression of heterologous secreted proteins are very common and well documented in the literature. For example, cell toxicity is one of the key problems in the expression of recombinant antibody fragments in bacteria (Hayhurst and Georgiou 2001, Lou *et al.* 2001).

The folding of proteins exported via the *sec* pathway occurs following translocation across the membrane. In both prokaryotes and in eukaryotes, the protein folding machinery in the secretory compartments (the periplasmic space in Gram negative bacteria and the endoplasmic reticulum in eucaryotic cells) is quite different from that of the cytoplasm (Wulfing and Pluckthun 1994, a Danesse and Silhavy 1998). Notably, the bacterial periplasmic space lacks highly sophisticated, ATP-dependent chaperone systems such as GroEL/GroES and the DnaK/DnaJ/GrpE network (Baneyx 1999). Proteins that depend on such chaperone systems for their folding frequently fail to reach their soluble, biologically active form following secretion into the bacterial periplasm and accumulate as inclusion bodies (Bowden *et al.* 1991).

Many enzymes require cofactors such as molybdopterin, iron-sulfur centers, FMN, FAD etc. that are synthesized in the cytoplasm and are incorporated into the protein during folding. If such proteins are secreted via the *sec* system, cofactor incorporation cannot occur since the protein is unfolded prior to export (Robinson 2000).

Moreover, the biosynthetic limitations inherent to the *sec* export machinery limit the functional diversity of combinatorial protein libraries (Lou *et al.* 2001, Hayhurst and Georgiou 2001, Arnold 2001). In the screening of combinatorial libraries, and in directed protein evolution in general, selected clones exhibiting a desired function are isolated based on their ability to pass though two "filters": a "biosynthetic filter" that eliminates proteins which are toxic to the cell or otherwise not compatible with expression, and a "function filter" which selects for the proteins having a desired function. In the screening of combinatorial libraries using display technologies (phage, bacterial, etc), a polypeptide must be secreted from the cytoplasm before it can be displayed on the surface of a biological particle such as filamentous phage or a microbial cell. However, many polypeptides are not compatible with export via the *sec* pathway or with folding within the bacterial periplasmic space. As a result, the polypeptide diversity of expression libraries is limited, among others, by the restrictions imposed by secretion. Bypassing these limitations will likely expand the diversity of polypeptide sequences in combinatorial libraries and thus, the ability to isolate novel binding proteins and enzymes.

The prior art is thus deficient in methods of directing efficient export of folded proteins from the cytoplasm of bacteria such as *E. coli.* In addition, the prior art has clearly demonstrated that many heterologous proteins, and in particular, human proteins, cannot be exported efficiently in bacteria. Deficiencies in methods for the folding and export of complex proteins often present a limitation in the isolation of proteins with desired function from combinatorial libraries screened by methods such as phage display. The present invention fulfills this long-standing need and desire in the art by providing methods of the secretion of complex polypeptides from bacteria and in particular proteins with multiple disulfide bonds through the Twin-Arginine Translocation pathway in bacteria. Moreover the present invention discloses methods for the screening of combinatorial polypeptide methods exported from the cytoplasm using the Twin=Arginine Translocation pathway.

### SUMMARY OF THE INVENTION

In one aspect, the invention provides a bacteria genetically transformed with an expression cassette comprising a leader peptide that directs protein export through the Twin Arginine Translocation pathway upstream of a gene encoding a heterologous polypeptide, wherein the bacteria comprises an oxidizing cytoplasm, and wherein the heterologous polypeptide is produced by the bacterial cell and comprises at least one disulfide bond. The bacteria may have an oxidizing cytoplasm. In certain embodiments of the invention, the heterologous polypeptide may contain from about 1 to about 17 disulfide bonds and/or may be produced in biologically-active form. In certain further embodiments of the invention, the leader peptide may be from a gene encoding a protein selected from the group consisting of *E. coli* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and YaeI. The leader peptide may also be derived from a gene encoding a homologue of any of these sequences.

In one embodiment of the invention, a bacteria provided may comprises one or more mutation that causes the bacteria to have an oxidizing cytoplasm. The bacteria may be a gram positive or gram negative bacteria. In one embodiment of the invention, the bacteria is *E. coli.* The heterologous polypeptide may be secreted from the bacteria and isolatable from the periplasm of the bacteria or an integral membrane protein The heterologous polypeptide may also be isolatable from a culture supernatant of the bacteria. In certain embodiments of the invention, the heterologous polypeptide is a mammalian polypeptide, for example, an antibody or fragment thereof. In a further embodiment, the heterologous polypeptide is selected from the group consisting of a polypeptide in native conformation, a mutated polypeptide and a truncated polypeptide. Such a heterologous polypeptide may be expressed on the surface of a cytoplasmic cell membrane of the bacteria, and/or on the surface of a periplasmic cell membrane of the bacteria and/or in the periplasm of the bacteria.

In yet another aspect, the invention provides a method of producing at least one heterologous polypeptide comprising at least one disulfide bond in a bacterial cell, comprising the steps of: a) constructing an expression cassette comprising a leader peptide that directs protein export through the Twin Arginine Translocation pathway upstream of a gene encoding a heterologous polypeptide; and b) expressing the expression cassette in a bacterial cell comprising an oxidizing cytoplasm, wherein the heterologous polypeptide is produced and comprises at least one disulfide bond. In one embodiment of the invention, the heterologous polypeptide contains from about 1 to about 17 disulfide bonds. The heterologous polypeptide may be produced in biologically-active form. Two of the heterologous polypeptides may be linked by at least one disulfide bond. In the method, the leader peptide may be from a gene encoding a protein selected from the group consisting of *E. coli* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and YaeI. In certain embodiments, a leader peptide of a homologues of such sequences is used.

In one embodiment of the invention, the heterologous polypeptide may be secreted from the bacterial cell. The heterologous polypeptide may be isolatable from the periplasm of the bacteria and/or may be an integral membrane protein. The heterologous polypeptide may also be isolatable from the culture supernatant of the bacterial cell. The heterologous polypeptide may be a mammalian polypeptide and may be selected from the group consisting of a polypeptide in native conformation, a mutated polypeptide and a truncated polypeptide. The heterologous polypeptide may be expressed on the surface of a cytoplasmic cell membrane of the bacteria, may be expressed on the surface of a periplasmic cell membrane of the bacteria, and may be an antibody or fragment thereof.

In yet another aspect, the invention provides a method of identifying a nucleic acid encoding a mutated polypeptide that can reconstitute protein oxidation in a secretory compartment, comprising the steps of: a) obtaining expression cassettes comprising a leader peptide specific for the Twin Arginine Translocation pathway upstream of nucleic acid sequences encoding mutated polypeptides of a protein with oxidizing activity; b) expressing the expression cassettes in bacteria that have oxidizing cytoplasm and impaired periplasmic disulfide bond formation; and c) selecting at least a first bacteria in which the impaired periplasmic disulfide bond formation activity has been complemented by expression of the expression cassette to identify a nucleic acid sequence encoding a mutated polypeptide that can reconstitute protein oxidation in a secretory compartment.

In still yet another aspect, the invention provides a method of screening a combinatorial library, comprising the steps of: a) generating a library of polypeptides of interest; b) constructing expression cassettes that place a leader peptide specific for the Twin Arginine Translocation pathway upstream of a gene encoding the polypeptides; c) expressing the expression cassettes in bacteria comprising an oxidizing cytoplasm; and d) screening for expressed secreted polypeptides. In the method, screening for expression may be by periplasmic expression, cytometric screening or phage display. The polypeptide may be a mammalian polypeptide and may be an antibody or fragment thereof. In certain embodiments of the invention, the leader peptide is derived from a gene encoding a protein selected from the group consisting of *E. coli,* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and YaeI. The leader peptide may also be from a homologues of these sequences.

### BRIEF DESCRIPTION OF THE DRAWINGS

So that the matter in which the above-recited features, advantages and objects of the invention as well as others which will become clear are attained and can be understood in detail, more particular descriptions and certain embodiments of the invention briefly summarized above are illustrated in the appended drawings. These drawings form a part of the specification. It is to be noted, however, that the appended drawings illustrate preferred embodiments of the invention and therefore are not to be considered limiting in their scope.

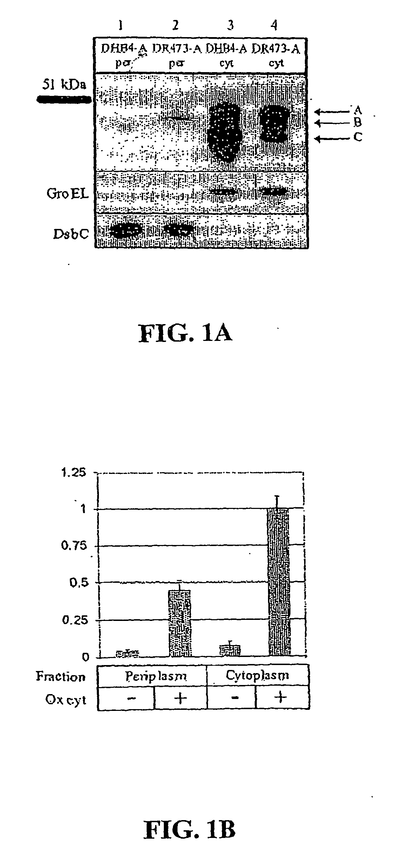

**FIG. 1A-B: FIG. 1A** shows a Western blot of periplasmic and cytoplasmic fractions from *E.Coli* cells expressing TorA-PhoA fusions in various strains. **FIG. 1B** shows the alkaline phosphatase activities detected in periplasmic fractions and in cytoplasmic fractions of strains DHB4-A (reducing cytoplasm denoted by -) or DR473-B Oxidizing cytoplasm denoted +).

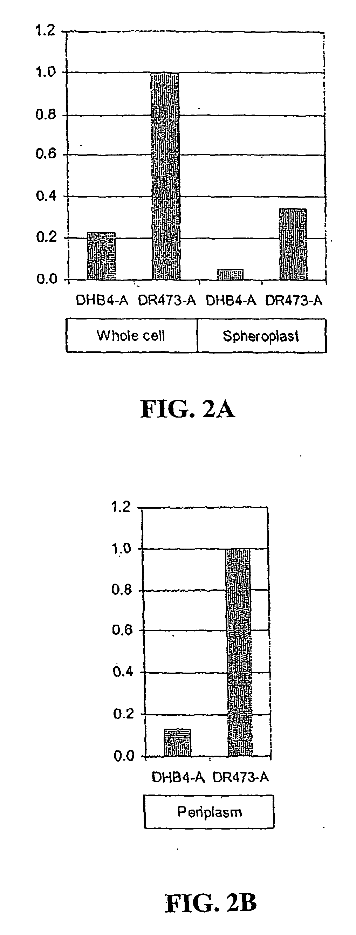

**FIG. 2A-B:** shows the distribution of vtPA activity in whole cell lysates, intact spheroplasts (**FIG. 2A** )or periplasmic fractions (**FIG. 2B**) upon expression of a TorA-vtPA fusion in either *E.Coli* DHB4-A (reducing cytoplasm) or in DR473-A (oxidizing cytoplasm).

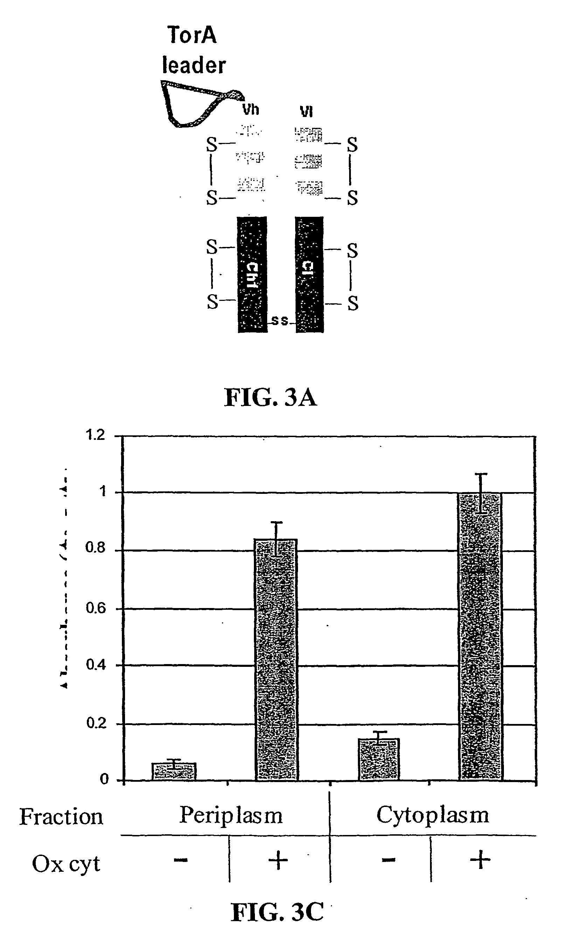

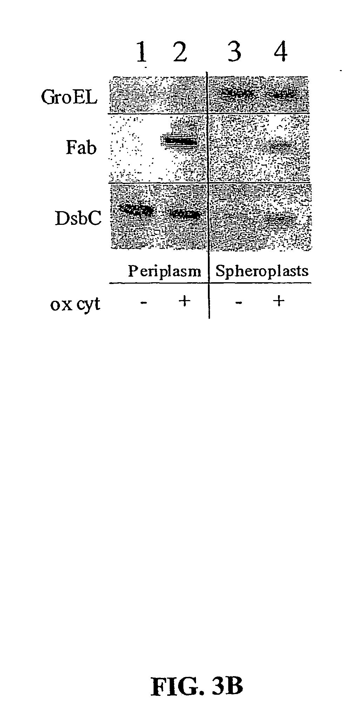

**FIG. 3A-C: FIG. 3A** shows a schematic of the TorA-Fab antibody fusion; **FIG. 3B** shows Western blot showing the distribution of Fab antibody protein in the periplasmic fraction or in the spheroplast fraction (cytoplasm) in strains having either an oxidizing (+) or reducing (-) cytoplasm (DR473-A or DHB4-A, respectively); **FIG. 3C** shows antibody binding activity for the samples shown in **FIG. 3B** as monitored by ELISA.

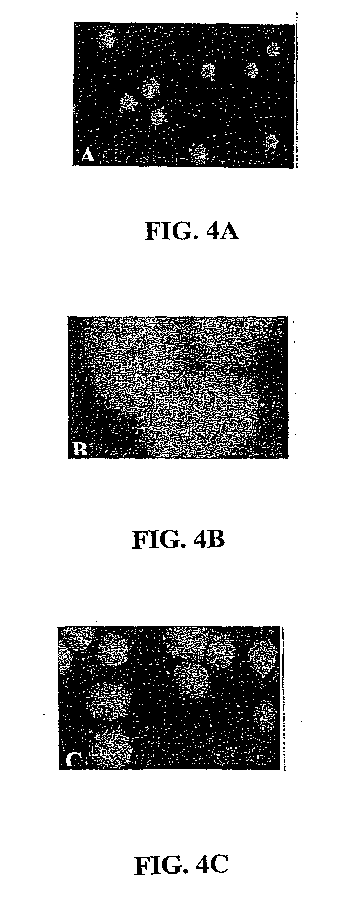

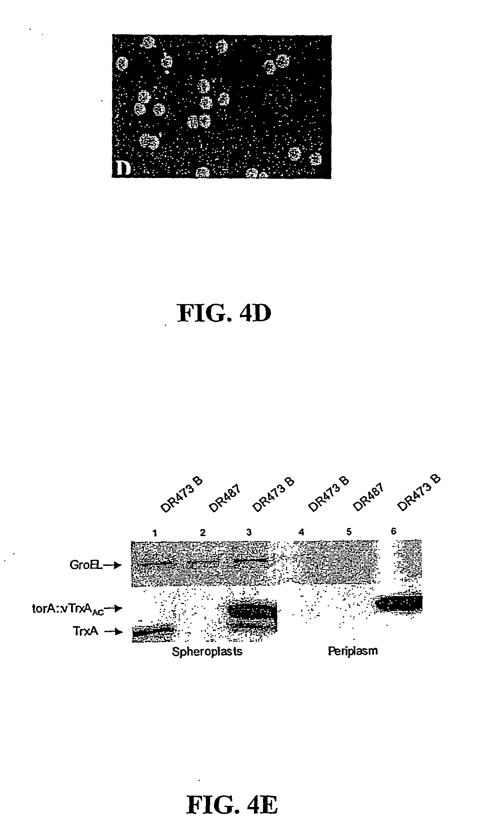

**FIG. 4A-E:** shows motility plates of cells: **(FIG. 4A)** DR473-B: **(FIG. 4B)** DR473; **(FIG. 4C)** DR473-B expressing the TorA-TrxA_{Ac} fusion; **(FIG. 4D)** MC1000 dsbB isogenic parent of DR473-B having a reducing cytoplasm; (**FIG. 4E**) Western blot showing the distribution of thioredoxin in the periplasm and cytoplasmic fractions in different strains.

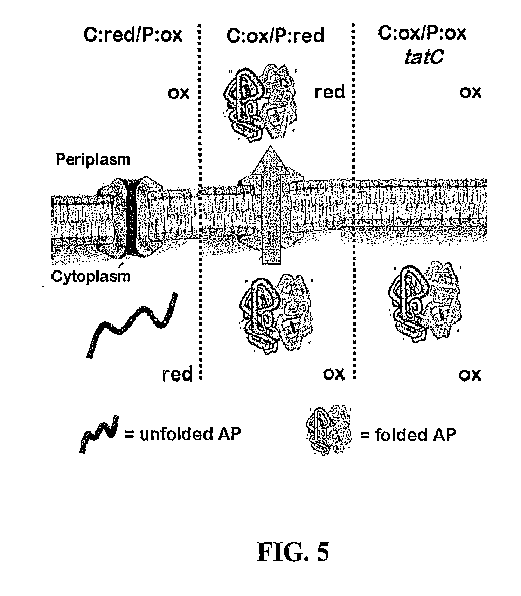

**FIG. 5** shows AP folding and export in different strain backgrounds. Ox and red refer to oxidizing and reducing redox potentials in the specified subcellular compartment. In C:ox (DR473) cells, AP is able to fold in the cytoplasm and thus can serve as a substrate for the Tat pathway. The localization of AP is not impaired in the C:ox/P:red strain DRA (DR473 *dsbA*). Deletion of *tatC* (or *tatB*) in the C:ox/P:ox strain blocks AP export.

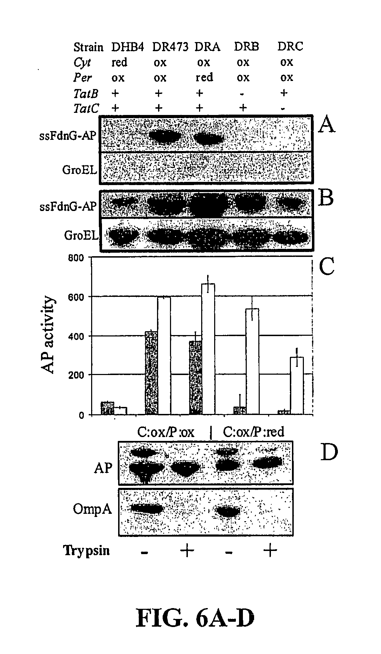

**FIG. 6A-D: FIG. 6A** shows subcellular localization of ssFdnG-AP. Immunoblotting of periplasmic (**FIG. 6A**) and cytoplasmic (**FIG. 6B**) fractions from cells expressing ssFdnG-AP. Samples were normalized based on the amount of total cell protein and resolved on 12% SDS-PAGE gels. GroEL was used as a fractionation marker by probing with anti-GroEL serum. **FIG. 6C:** AP activity for the same periplasmic (solid) and cytoplasmic (empty) fractions as in A and B. **FIG. 6D:** Trypsin sensitivity analysis of periplasmic fractions collected from C:ox/P:ox and C:ox/P:red cells. Samples were separated on 4-20% SDS-PAGE gels and probed with anti-AP and anti-OmpA serum.

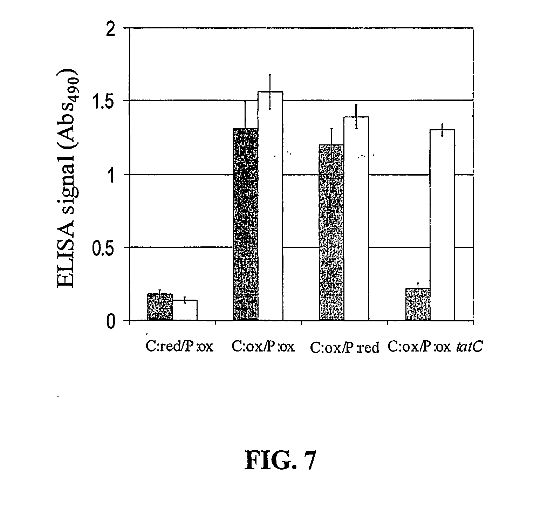

**FIG. 7** shows Tat export of a scFv antibody. Detection of scFv by ELISA in the periplasmic and cytoplasmic fractions. Periplasmic (solid) and cytoplasmic (empty) samples were serially diluted and started from the same amount of total protein. Data reported is from a 4-fold dilution and is the average of two independent studies.

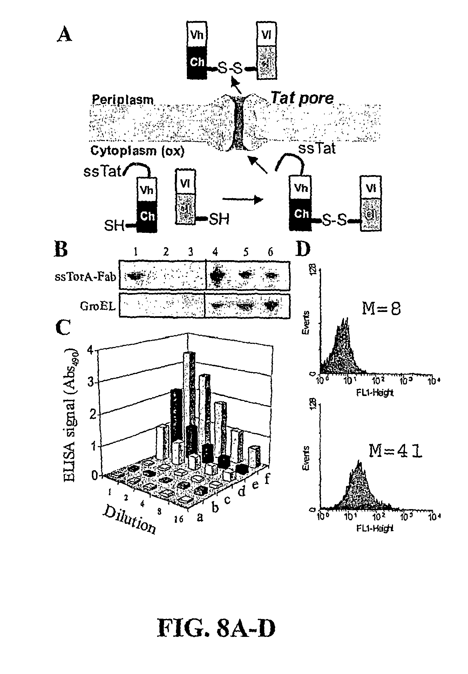

**FIG. 8A-D: FIG. 8A** shows Tat export of an anti-digoxin antibody fragment (F_{AB}). Tat export of F_{AB} antibodies. **FIG. 8B:** Western blotting of periplasmic (lanes 1-3) and cytoplasmic (lanes 4-6) fractions collected from cells co-expressing TorA-Fab fusion and ΔssDsbC. Strains used were: (1,4) C:ox/P:ox; (2,5) C:ox/P:ox/*tatC* and (3,6) C:red/P:ox. All lanes were loaded with the same amount of total protein and anti-mouse IgG F(ab')₂ antibody was used to detect the F_{AB} light chain. GroEL was used as a fractionation marker for the spheroplast fractions. **FIG. 8C:** ELISA of periplasmic (a,c,e) and cytoplasmic (b,d,f) fractions collected from (a,b) C:red/P:ox; (c,d) C:ox/P:ox/*tatC;* and (e,f) C:ox/P:ox cells. **FIG. 8D:** Flow cytometric analysis of C:red/P:ox cells (top) and C:ox/P:ox cells (bottom) cells expressing TorA-Fab and ΔssDsbC and labeled with FITC-digoxin.

### DETAILED DESCRIPTION OF THE INVENTION

To examine the relationship between folding and export competence via the twin-arginine translocation (Tat) pathway, the inventors analyzed the subcellular localization of fusions between a set of 8 putative Tat leader peptides and alkaline phosphatase in isogenic *E. coli* strains that either allow or disfavor the formation of protein disulfide bonds in the cytoplasm. It was shown that export via the Tat translocator is observed only in strains that enable oxidative protein folding in the cytoplasm. Further, it was shown that other disulfide-containing proteins, namely single chain Fv and heterodimeric F_{AB} antibody fragments, are export-competent only in strains having an oxidizing cytoplasm. Notably, functional, heterodimeric F_{AB} protein was exported from the cytoplasm by means of a Tat leader peptide fused to the heavy chain alone, indicating that the formation of a disulfide bonded dimer precedes export. These results demonstrate that *in vivo* only proteins that have attained the native conformation are exported via the Tat translocator, indicating that a folding quality control mechanism is intrinsic to the export process. The ability to export proteins with disulfide bonds and also the folding proofing feature of the Tat pathway have many applications.

The findings of the inventors have many applications. It was demonstrated that proteins with structural disulfide bonds can be efficiently exported by the Tat pathway. Many industrially important secreted proteins including enzymes contain multiple disulfide bonds that are required for proper folding. Such proteins may, for example, be secreted via the Tat pathway in bacteria with oxidizing cytoplasm, such as the C:ox strains. The advantage of utilizing the Tat pathway is that it may alleviate problems with cell toxicity often encountered when proteins exported by the Sec pathway become 'stuck' in the translocon. Second, the folding quality control feature of the Tat pathway may be exploited in combinatorial library screening studies to "weed out" mutant polypeptides that cannot fold properly.

The Tat pathway was discovered less than five years ago as a new mechanism for protein secretion, first in the thylakoid membranes of photosynthetic organisms and subsequently in bacteria (Settles *et al.,* 1997; Weiner *et al.,* 1998). This secretion mechanism has been named the "Twin Arginine Translocation" or TAT pathway because of the signature Arg-Arg motif found in the leader peptides of proteins that are engaged in this mode of export. Estimates based on proteomics and bioinformatics analyses indicate that 5-8% of the secreted proteins in bacteria such as *E. coli* or *B. subtilis* are translocated via the TAT pathway (Berks *et al.* 2000, Robinson and Bolhuis 2001). The main features of this pathway, which is still very poorly understood, are described below.

First and foremost, whereas polypeptides exported by the *sec* pathway and other secretion mechanisms thread the membrane in an unfolded form and reach their native conformation after export is complete, proteins secreted via the TAT pathway first fold in the- cytoplasm and are then translocated across the membrane in a compact, native-like state. In fact, for this reason the TAT pathway is responsible for the export of proteins that require the incorporation of cofactors or the assembly of different subunits in the cytoplasm (Rodrigue *et al.* 1999, Robinson 2000, Sanders *et al.* 2001). Secretion does not occur through the SecYEG translocon but rather through a presumably larger diameter pore formed by the TatABC and E proteins (Sargent *et al.* 2001). Large proteins of molecular weight up to at least 120 kDa have been documented to b e exported though the TAT pathway. Moreover, secretion through the TAT pathway is not linked to the hydrolysis of ATP but instead is driven by the asymmetric distribution of protons across the membrane, orΔpH.

The leader peptides required for targeting proteins to the TAT apparatus are longer and less hydrophobic than *sec*-specific leaders. TAT-specific leader peptides are on average 14 amino acids longer due to an extended amino terminal region and more basic residues in the c-region (Cristobal *et al.,* 1999). However, the hydrophobic region in the TAT-specific leader peptides is significantly shorter due to a higher occurrence of glycine and threonine residues. A hallmark of both plant and prokaryotic TAT-specific leader peptides is the presence of the distinctive and conserved (S/T)-R-R-x-F-L-K (SEQ ID No. 9) sequence motif which is absent from *sec-*specific leader peptides (Robinson and Bolhuis, 2001). This sequence motif is located at the amino terminal region/hydrophobic core boundary within leader peptides of known and predicted TAT substrates (Berks, Mol 1996). Mutation of either arginine residue within the signal peptide significantly reduces the efficiency of protein translocation (Cristobal *et al.,* 1999).

The twin-arginine (RR) motifs of wheat pre-23K and pre-Hcf136 are essential for targeting by the thylakoid TAT pathway. This motif is a central feature of TAT leader peptide in general. Bacterial twin-arginine-signal peptides are similar to thylakoid TAT signals and can direct TAT-dependent targeting into plant thylakoids with high efficiency. However, the vast majority of bacterial signal peptides contain conserved sequence elements in addition to the twin-arginine motif that imply special functions. There is a heavy bias towards phenylalanine at the second position after the twin-arginine motif, and many of the signals contain lysine at the fourth position. None of the known thylakoid twin-arginine signals contains phenylalanine at this position and only one (*Arabidopsis* P29) contains lysine as the fourth residue after the twin-arginine motif. The precise roles of these highly conserved features are unclear. The phenylalanine residue can be replaced by Leu but not by Ala without undue effects, which indicates that hydrophobicity, rather than the phenylalanine side-chain, might be the important determinant. Similarly, replacement of the Lys residue does not impede export (Robinson and Bolhuis, 2001).

Due to the novelty of the TAT export system, it has not yet been exploited for protein expression or for any other biotechnology applications. However, the present invention demonstrates that the TAT pathway offers the potential to overcome the limitations associated with the existing technology for protein secretion in bacteria (*i.e*. via the *sec* pathway). Proteins exported via the TAT pathway fold in the cytoplasm which provides a more extensive machinery for assisting the formation of native protein structure as compared to the periplasm which contains a limited array of folding accessory factors. Importantly, as was mentioned above, the TAT pathway represents the only physiological route for the export of complex proteins containing cofactors or consisting of multiple subunits which must associate during folding and therefore cannot be exported individually via the *sec* pathway (Robinson 2000). Perhaps because of the large diameter of the TatABC translocation complex, there are no known instances where export via the TAT pathway has led to led proteins becoming stuck in the channel or cell toxicity effects. Whereas secretion through the *sec* pathway results in the consumption of ATP, TAT export is dependent only on ΔpH and therefore is less energetically draining for the cell (Musser and Theg 2000). Finally, the different folding considerations and lower toxicity associated with TAT-specific export suggest that the utilization of the TAT pathway for protein display can expand the diversity of expressed polypeptides in combinatorial libraries, a fact that in turn should translate into isolation of functionally superior protein mutants.

The export of proteins that normally contain disulfide bonds via the TAT pathway presents a problem. The TAT pathway normally accepts as substrates proteins that are already folded. However, because the cytoplasm is highly reducing, proteins that contain disulfide bonds in their native state cannot fold and therefore cannot be accepted as substrates for export via the TAT pathway. Indeed, extensive earlier studies have demonstrated that proteins requiring disulfide bonds for folding are not exported via the TAT pathway (Stanley *et al.,* 2002).

Hence, there is a need to change the cytoplasm into an oxidizing state for secretion through the TAT pathway. Normally, the bacterial cytoplasm is maintained in a reduced state due to the presence of reducing components such as glutathione and thioredoxins that strongly disfavor the formation of disulfide bonds within proteins (Ritz and Beckwith, 2001). Earlier work by Bessette *et al.* resulted in the engineering of bacterial strains having a highly oxidizing cytoplasm that allows efficient formation of disulfide bonds (Bessette *et al.,* 1999).

As shown in Bessette *et al., E. coli* depends on aerobic growth in the presence of either of the two major thiol reduction systems: the thioredoxin and the glutathione-glutaredoxin pathways. Both the thioredoxins and the glutaredoxins are maintained in a reduced state by the action of thioredoxin reductase (TrxB) and glutathione, respectively. Glutathione is synthesized by the *gshA* and *gshB* gene products. The enzyme glutathione oxidoreductase, the product of the *gor* gene, is required to reduce oxidized glutathione and complete the catalytic cycle of the glutathione-glutaredoxin system. When both of these thiol reduction pathways were eliminated by mutation in a *trxB gor* or *trxB gshA* double mutant, the cells grew extremely slowly. However, these cells can be rescued by the addition of the reductant DTT to the growth medium. When a *trxB gor* or *trxB gshA* strain was grown in media containing DTT and then transferred to medium lacking DTT, the cytoplasm became even more oxidizing than in the *trxB* strain. Even when grown in the presence of DTT, both the *trxB gshA* and *trxb gor* strains gave rise to fast growing derivatives at a high frequency. Because the *trxB, gshA,* and *gor* alleles in these strains are nonreverting null mutations, the faster-growing derivatives must result from extragenic suppressor mutations.

Subsequently, it was determined that these faster-growing derivatives accumulated suppressor mutations in the alkyl hydroperoxidase (ahpC) gene. The resulting *ahpC** allele allows efficient growth in normal (non-reducing) media without compromising the formation of disulfide bonds in the cytoplasm. Thus *trxB, gor ahpC** mutant strains (such as *E*. *coli* DR473 or FA113) exhibit the ability to support disulfide bond formation in the cytoplasm, and also can grow equally well as the corresponding wild-type strain DHB4 in both rich and minimal media. In the studies described herein, the *E coli* strain DR473-A also contains a mutation that inactivates the periplasmic oxidase *dsbA.* In addition, DR473-B is a *dsbB* derivative of DR473. DsbB normally serves as the oxidant of DsbA, and mutants deficient in either enzyme are impaired in the oxidation of proteins in the periplasmic space. Such mutants can form disulfides in the cytoplasm but not in the periplasm.

Disclosed herein are transcriptional fusions between the TAT-specific signal sequence of *E. coli* trimethylamine *N*-oxide reductase (TorA) and various genes encoding multidisulfide proteins. These proteins include: (a) *E. coli* alkaline phosphatase (PhoA) containing two disulfide bonds that are consecutive in the primary sequence (PhoA is a common reporter for *sec* pathway secretion a s well as disulfide bond formation *in vivo*); (b) an anti-digoxin antibody fragment (Fab) containing four intra- and one inter-molecular disulfide bond; (c) a truncated version of human tissue plasminogen activator (vtPA) consisting of the kringle 2 and protease domains with a total of nine disulfide linkages; and (d) variants of the *E. coli* thioredoxin (TrxA). It is shown that the latter protein when expressed in the cytoplasm of the oxidizing strain DR473 and exported via the TAT pathway can serve as a general oxidant within the periplasm. In this case, TrxA that has been pre-oxidized in the cytoplasm and secreted into the periplasm via the TAT pathway can complement *dsbB* mutants deficient in the normal pathway for the formation of disulfide bonds in the periplasmic space. The export of pre-oxidized thioredoxin variants serves to provide oxidants in the bacterial periplasm and to thus restore defects caused by the lack of normal periplasmic bacterial oxidants such as the enzymes DsbA and DsbB. Defects of *dsbA* or *dsbB* mutants that are restored by the oxidized thioredoxin include cell motility, growth in minimal media and infectivity by filamentous phages.

In one aspect of the present invention, there is provided a method for producing biologically-active heterologous polypeptide containing multiple disulfide bonds in a bacterial cell. A heterologous polypeptide produced by this method may contain up to 17 disulfide bonds, and the polypeptide can either be secreted from the bacteria. Furthermore, the polypeptide may be isolated from the periplasm, the culture supernatant of the bacterial cell, or is an integral membrane protein. The method involves first constructing an expression cassette that places a leader peptide that directs protein export through the Twin Arginine Translocation pathway upstream of a gene encoding the heterologous polypeptide, and then expressing the heterologous polypeptide in bacteria such a s *E*. *coli* strain FA113 or *E. coli* strain DR473 that have an oxidizing cytoplasm.

Methods which are well known to those skilled in the art can be used to construct expression cassettes or vectors containing appropriate transcriptional and translational control signals. See, for example, the techniques described in Sambrook *et al.,* 1989, *Molecular Cloning: A Laboratory Manual* (2nd Ed.), Cold Spring Harbor Press, N.Y. Vectors of the invention include, but are not limited to, plasmid vectors, viral vectors and chromosomal integration vectors.

The secretion method disclosed herein is applicable to numerous eukaryotic proteins of importance to the pharmaceutical and bioprocessing industries. Currently, the production of technologically important proteins with four or more disulfide bonds is costly and complicated, and must rely either on expression in higher eukaryotes that provide a favorable environment for the formation of disulfide bonds, or refolding from inclusion bodies (Hockney, 1994; Georgiou and Valax, 1996). For example, tissue plasminogen activator (tPA) is currently produced in bacteria inclusion bodies. In typical procedures, the proteins are released from inclusion bodies using a variety of chaotropic agents, then isolated and refolded by employing reducing agents. Generally, refolding results in low yields of biologically active material.

The process of secretion disclosed herein provides an efficient method of producing on an economic scale complex eukaryotic proteins with multiple disulfide bonds. Moreover, the proteins produced by the method disclosed herein are correctly fully folded and biologically active without the need for reactivation or subsequent processing once isolated from a host cell. As used herein, the biologically active molecules generated by the disclosed methods are typically molecules with 4 or more disulfide bonds, although biologically active molecules could be generated having 17 or more disulfide bonds. These disulfide bonds form from specific orientations to promote correct folding of the native protein. Multiple disulfide bonds resulting from improper orientation of nascently formed proteins would lead to misfolding and loss or absence of biological activity. Using the methods described by the present invention, a biologically-active polypeptide containing multiple disulfide bonds is folded correctly; disulfide bonds will form to provide a tertiary and where applicable, quaternary structure leading to a molecule with native functional activity with respect to substrates and/or catalytic properties.

One immediate problem solved by the methods of the present invention is that proteins with multiple disulfide bonds can now be exported to the periplasm in a folded and therefore active conformation. Complex proteins containing multiple disulfide bonds can be folded in the cytoplasm with the assistance of a full complement of folding accessory factors that facilitate nascent polypeptides in reaching their native conformation. The folded proteins are then secreted into the periplasmic space or the growth medium in a functional form, thus alleviating problems associated with inclusion bodies and simplifying recovery. In addition, active recombinant proteins accumulate simultaneously in two bacterial compartments (cytoplasm and periplasm), leading to greater overall yields of numerous complex proteins which previously could not actively accumulate in both compartments concurrently.

In another aspect of the present invention, there is provided a method of identifying a mutated polypeptide that can reconstitute protein oxidation in a secretory compartment. The method involves constructing expression cassettes that place a leader peptide specific for the Twin Arginine Translocation pathway upstream of a library of mutated polypeptides encoding a protein with oxidizing activity. The expression cassettes are then expressed in bacteria that have oxidizing cytoplasm and an impaired periplasmic disulfide bond formation pathway. Mutated polypeptide that can reconstitute protein oxidation are isolated from bacteria that express an activity resulted from periplasmic disulfide bond formation. In one embodiment, a representative bacteria that have oxidizing cytoplasm and an impaired periplasmic disulfide bond formation pathway is *E. coli* DR473-A or *E. coli* DR473-B, and the mutated polypeptides encode a mutated thioredoxin 1 (TrxA).

Moreover, the present invention of TAT secretion can be exploited for uses in combinatorial library screening. Currently, protein display technologies for library screening hinge on the export of the polypeptide chains from the cytoplasm. So far, all *E. coli* display technologies such as phage display, bacterial display, periplasmic expression and cytometric screening (PECS) utilize the *sec* pathway to achieve protein secretion. However, because of the limitations inherent to secretion via the *sec* pathway, only a fraction of genes in displayed libraries actually express proteins in a functional form. For example, polypeptide sequences that become stuck in the *sec* pore fold too fast within the cytoplasm, or require association with cofactors or participation of cytoplasmic chaperones cannot be properly expressed and are therefore eliminated from the library. The biosynthetic restrictions imposed by the requirement for export via the *sec* pathway may be eliminated, or at the very least alleviated, by employing the Tat pathway. This is likely to increase the diversity of expressed sequences in a library, which in turn will facilitate the isolation of novel proteins with desired functional properties.

Export via the Tat pathway can be utilized in conjunction with two protein library screening methodologies: phage display and PECS (periplasmic expression and cytometric screening). The former currently represents the most widely used format for protein library screening. Periplasmic expression and cytometric screening, on the other hand, is the only library screening methodology in which proteins are expressed in soluble form in the bacterial periplasm and are not tethered to a biological surface (Chen *et al.* 2001). Periplasmic expression and cytometric screening is a powerful and rapid technology for isolating ligand-binding proteins from diverse libraries. Briefly, *E. coli* cells expressing a library of proteins secreted into the periplasmic space are incubated with a fluorescent conjugate of the target ligand. Under the proper incubation conditions, ligands as large as 12 kDa equilibrate within the periplasmic space, without leakage of proteins or compromise of the cell's viability. Thus, the bacterial cell envelope effectively serves as a dialysis bag to selectively retain protein:fluorescent-ligand complexes but not free ligand. Cells displaying increased fluorescence are then isolated by flow cytometry (Chen *et al.* 2001).

Since the only requirement for periplasmic expression and cytometric screening is that proteins are localized in the periplasm, utilizing the Tat pathway for export instead of the *sec* pathway is straightforward. On the other hand, changing the secretion pathway in conjunction with protein display on phage is more complicated. Proteins are displayed on filamentous phage as fusions to the phage protein p3. Fusions to p3 are secreted from the cytoplasm, typically using the *sec*-specific *pe1B* leader sequence (Hoogenboom *et al.* 1998). Following secretion, the p3 fusion proteins reside in the cytoplasmic membrane (on the periplasmic side) (Endeman and Model 1995) and then are incorporated into the nascent phage particle which is eventually extruded from the cell though a pore in the outer membrane (Russell 1998). The p3 protein contains 4 disulfide bonds which are required for proper folding and are normally formed after the protein has been secreted in the periplasmic space (Kremser and Rashed 1994). However, the Tat pathway exports proteins that have already attained a native-like, compact conformation in the cytoplasm. Therefore, in order to switch the secretion pathway utilized for phage display, it is necessary not only to append a Tat-specific leader peptide at the N-terminal of the p3 fusion but also to use a strain having an oxidizing cytoplasm. As described above, oxidative folding in the cytoplasm of a *trxB gor ahpC* mutant strain such as FA113 is a requirement for the Tat-specific export of proteins with disulfide bonds.

Thus, in yet another aspect of the present invention, there is provided a method of screening a combinatorial library that involves constructing expression cassettes comprising a leader peptide specific for the Twin Arginine Translocation pathway upstream of a library of polypeptides of interest. The library is then screened by expressing and secreting the polypeptides through the TAT pathway. In general, the library can be expressed in bacteria such as *E. coli* strain FA113 or *E. coli* strain DR473, and the library can be screened by the method of periplasmic expression and cytometric screening or other screening methods well known to those of ordinary skill in the art.

As used herein, "polypeptide" or "polypeptide of interest" refers generally to peptides and proteins having more than about ten amino acids. The polypeptides are "heterologous," meaning that they are foreign to the host cell being utilized, such as a human protein produced by a CHO cell, or a yeast polypeptide produced by a mammalian cell, or a human polypeptide produced from a human cell line that is not the native source of the polypeptide. Examples of a polypeptide of interest include, but are not limited to, molecules such as renin, a growth hormone (including human growth hormone), bovine growth hormone, growth hormone releasing factor, parathyroid hormone, thyroid stimulating hormone, lipoproteins, α1-antitrypsin, insulin A-chain, insulin β-chain, proinsulin, thrombopoietin, follicle stimulating hormone, calcitonin, luteinizing hormone, glucagon, clotting factors (such as factor VIIIC, factor IX, tissue factor, and von Willebrands factor), anti-clotting factors (such as Protein C, atrial naturietic factor, lung surfactant), a plasminogen activator, (such as human tPA or urokinase), mammalian trypsin inhibitor, brain-derived neurotrophic growth factor, kallikreins, CTNF, gp 120, anti-HER-2, human chorionic gonadotropin, mammalian pancreatic trypsin inhibitor, antibodies, antibody fragments, protease inhibitors, therapeutic enzymes, lymphokines, cytokines, growth factors, neurotrophic factors, insulin chains or pro-insulin, immunotoxins, bombesin, thrombin, tumor necrosis factor-α or β, enkephalinase, a serum albumin (such as human serum albumin), mullerian-inhibiting substance, relaxin A-chain, relaxin B-chain, prorelaxin, mouse gonadotropin-associated peptide, a microbial protein (such as β-lactamase), Dnase, inhibin, activin, vascular endothelial growth factor (VEGF), receptors for hormones or growth factors, integrin, protein A or D, rheumatoid factors, neurotrophic factors (such as neurotrophin-3, -4, -5, or -6), or a nerve growth factor (such as NGF⁻β), cardiotrophins (cardiac hypertrophy factor) (such as cardiotrophin-1), platelet-derived growth factor (PDGF), fibroblast growth factor (such as α FGF and β FGF), epidermal growth factor (EGF), transforming growth factor (TGF) (such as TGF-α, TGF-β1, TGF-β2, TGF-β3, TGF-β4, or TGF-β5), insulin-like growth factor-I and insulin-like growth factor-II, des-IGF-I (brain IGF-I), insulin-like growth factor binding proteins, CD proteins (such as CD-3, CD-4, CD-8, and CD-19), erythropoietin, osteoinductive factors, bone morphogenetic proteins (BMPs), interferons (such as interferon-α interferon-β, and interferon-γ), colony stimulating factors (CSFs) (*e.g.,* M-CSF, GM-CSF, and G-CSF), interleukins (Ils) (such as IL-1 to IL-10), superoxide dismutase, T-cell receptors, surface membrane proteins, decay accelerating factor, viral antigens such as a portion of the AIDS envelope, transport proteins, homing receptors, addressins, regulatory proteins, antigens such as gp 120(IIIb), or derivatives or active fragments of any of the peptides listed above. The polypeptides may be native or mutated polypeptides, and preferred sources for such mammalian polypeptides include human, bovine, equine, porcine, lupine, and rodent sources, with human proteins being particularly preferred. The following examples are given for the purpose of illustrating various embodiments of the invention and are not meant to limit the present invention in any fashion.

### EXAMPLE 1

### PLASMIDS AND LIBRARY CONSTRUCTION

*E. coli* thioredoxin (TrxA) expression plasmids were constructed as follows. Wild type thioredoxin 1 and thioredoxin 1 with the dsbA active site (trx-A_{dsbA}, -CPHC-) were amplified by PCR from plasmids pFA3 and pFA6 respectively (Mossner *et al.,* 1999) with primers Sall-TrxA-F (5'-ctctcgtcgacatgagcgataaaattattcac-3' (SEQ ID NO:1) and R-TrxA-Hind3 (5'-ctctcaagcttacgccaggttagcgtcgag-3'(SEQ ID NO:2). These primers introduce SalI and HindIII restriction sites at the 5' and 3' ends of the TrxA gene respectively. PCR amplified fragments were cloned into pBAD33-TorA plasmid (signal sequence (first 150 base pairs) of trimethylamine N-oxide reductase (TorA) cloned into pBAD33 (Guzman *et al.,* 1995) at SalI / HindIII sites, obtaining pBAD33-TorA::TrxA and pBAD33-TorA::TrxA_{dsbA}. Using these two plasmids as a template, the TorA::TrxA and TorA::TrxA_{dsbA} inserts were amplified by PCR with primers that introduce BspHI (NcoI compatible) and HindIII restriction sites at the 5' and 3' ends respectively. These PCR amplified fragments were cloned into pTrc99a (Amersham Pharmacia) at NcoI / HindIII sites, obtaining pTrc99a-TorA::TrxA and pTrc99a-TorA::TrxA_{dsbA}.

A TrxA-CXXC- library was constructed as follows. An overlap PCR strategy using a randomized oligonucleotide primer was used to construct the TrxA library with randomized amino acids at the positions 34 and 35 (-CXXC- motif). Using pBAD33-TorA::TrXA as template, the N terminal part of TrxA was amplified with primers Sall-trxA-F (5'-ctctcgtcgacatgagcgataaaattattcac-3' (SEQ ID NO:3) and N-ovlpTrxA-R (5'-ctctgcccagaaatcgacga-3' (SEQ ID NO:4)), whereas the C terminal part was amplified with the mutagenic primer TrxA-CXXC-F (5'-tcgtcgatttctgggcagagtggtgcNNNNNNtgcaaaatgatcgccccgat-3' (SEQ ID NO:5)) and R-TrxA-Hindi (5'-ctctcaagcttacgccaggttagcgtcgag-3' (SEQ ID NO:6). Both PCR amplified fragments were used as a template for an overlap PCR with Sall-trxA-F and R-TrxA-Hind3 primers. The product of this overlap PCR was cloned into pTrc99a-TorA::TrxA at SalI / HindIII sites after removal of the segment coding for wild type TrxA, this gives the pTrc99a-TorA::TrxA_{cxxc} plasmid library. The pTrc99a-TorA::TrxA_{cxxc} plasmid library was electroportated into DR473 dsbB and plated on Luria-Bertani (LB) media plates with 100 µg of ampicillin per ml.

### EXAMPLE 2

### MOTILITY ASSAY

A single colony was first grown overnight in M9 casein media (1X M9 minimal salts (Sigma, M6030), 0.4% (w/v) glycerol, 0.1% (w/v) casein enzymatic hydrolyzate (Sigma, C0626), 2 mM MgSO₄, 0.05. mg/ml thiamine) with the correspondent antibiotic at 37°C with shaking. The overnight culture was diluted based on OD₆₀₀, so approximately 100 cells were platted on an M9_{casein} motility plate (M9_{casein} media with 0.3 % (w/v) agar plus adequate antibiotic). Cells were then grown at 37°C for 40 hours.

To screen for a clone in the *TrxA-*CXXC library that has the ability to restore motility, approximately 2000 ampicillin-resistant colonies were pooled and grown overnight in M9_{casein} media at 37°C with shaking. The overnight culture was diluted (2 × 10⁻⁶) and plated on M9_{casein} motility plates with 100 µg/ml of ampicillin. After growing at 37°C. for 36 hours, approximately 3600 ampicillin-resistant colonies were obtained. Single colonies were selected based on the presence and size of motility 'halos.'

### EXAMPLE 3

### TAT-SPECIFIC EXPORT OF ALKALINE PHOSPHATASE (PHOA)

The *E. coli* alkaline phosphatase (PhoA) gene was fused to the TorA leader peptide using the expression vector pBAD33-TorA. The resulting TorA-PhoA fusion protein was exported to the periplasm when produced in a strain with an oxidizing cytoplasm (strain DR473-A, FIG. 1A, lane 2). In contrast, there was no measurable PhoA protein in the periplasmic compartment of the isogenic parental strain (strain DHB4-A) having a reducing cytoplasm (FIG. 1A, lane 1). Importantly, large quantities of PhoA protein were synthesized and accumulated in the cytoplasm of both DHB4-A and DR473-A cells. However, the reducing cytoplasm of DHB4-A prevents proper folding of PhoA as evidenced by a complete lack of activity in both the cytoplasmic and periplasmic compartments (FIG. 1B). The improper folding of PhoA in the cytoplasm of DHB4-A appears to have two distinct outcomes: i) there was significant degradation of the misfolded PhoA protein in the cytoplasm of DHB4-A cells as evidenced by the numerous lower molecular weight bands on the Western blot (FIG. 1A, lane 3); and ii) the lack of oxidative folding in the cytoplasm rendered PhoA incompatible with TAT-dependent secretion such that no measurable PhoA protein (FIG. 1A) or alkaline phosphatase activity (FIG. 1B) were observed in the DHB4 periplasmic fractions.

In the above studies, both the DHB4-A and DR473-A mutants were completely unable to oxidize periplasmic proteins due to mutation in the periplasmic oxidant *dsbA.* Therefore formation of the disulfide bonds enabling PhoA activity in DR473-A cells can only occur in the cytoplasm. These disulfides, in turn, were maintained intact during export through the TAT pathway.

In FIG. 1A, the A arrow points to the precursor protein having an intact TorA signal peptide, B is the mature protein having the TorA signal peptide removed by signal peptidase, and C are lower molecular weight degradation products. GroEL and DsbC were used as cytoplasmic and periplasmic fractionation markers, respectively. The observation that GroEL was predominantly in the cytoplasm fraction and DsbC was predominantly in the periplasmic fraction indicates that the subcellular fractionations were successfully performed.

### EXAMPLE 4

### TAT-SPECIFIC EXPORT OF TRUNCATED VERSION OF HUMAN TISSUE PLASMINOGEN ACTIVATOR (VTPA)

Similar studies as outlined above for PhoA were performed for a truncated variant of human tissue plasminogen activator (vtPA) expressed as a fusion to the TorA leader peptide. A gene encoding a truncated variant of tpA (Bessette *et al.* 2001) was fused to the sequence encoding the TorA leader in pBAD33-TorA. As shown in FIG. 2, when the TorA-vtPA fusion protein was expressed in cells with a reducing cytoplasm (DHB4-A), very low levels of tissue plasminogen activity were observed in whole cell lysates. The majority of this activity fractionated to the periplasmic compartment (FIG. 2B), suggesting that even in the reducing cytoplasm of DHB4-A, a small amount of vtPA was properly folded and thus can be exported by the TAT pathway. Remarkably, very high levels of tPA activity were observed in whole cell lysates when the same TorA-vtPA construct was produced in the oxidizing cytoplasm of DR473 cells (FIG. 2A). Upon subcellular fractionations, the majority of the tPA enzymatic activity was localized to the periplasm. Notably, the tPA activity in the periplasmic fractions of DR473 cells was extremely high relative to the activity observed for DHB4 cells (FIG. 2B).

### EXAMPLE 5

### TAT-SPECIFIC EXPORT OF AN ANTI-DIGOXIN ANTIBODY FRAGMENT

The heavy chain of an antibody specific for digoxin (Levis *et al.* 2001) was fused in frame to the TorA leader peptide in the pBAD33-TorA expression vector containing the arabinose inducible promoter. A schematic of the fusion is shown in FIG. 3A. Of note, the antigen-binding fragment (Fab) of the antibody contains five total disulfide bonds: four intramolecular bridges and one intermolecular disulfide bond covalently linking the variable and constant regions of the heavy chain to the variable and constant regions of the light chain of the Fab. One unique feature of this construct is that the TAT export signal was appended only to the variable region of the heavy chain. Therefore, periplasmic accumulation of active Fab required recruitment of the light chain by the TorA-heavy chain fusion prior to export. In this fashion, the TorA-heavy chain carries the light chain into the periplasm in a 'piggyback' fashion only if the interchain disulfide bridge is formed first in the cytoplasm.

As was the case for PhoA and vtPA, it was observed that expression of the TorA-Fab fusion protein in cells with a reducing cytoplasm (DHB4-A) resulted in virtually no detectable Fab in either the periplasmic or cytoplasmic fractions (FIG. 3B, lanes 1 and 3). In contrast, expression of the same construct in cells with an oxidizing cytoplasm (DR473-A) resulted in accumulation of the Fab in both periplasmic and cytoplasmic fractions (FIG. 3B, lanes 2 and 4). Of note, the majority of the Fab protein as detected by Western blot analysis was localized to the periplasmic compartment. Importantly, the presence of the Fab antibody was probed using a primary antibody that recognizes mouse light chain sequences. Therefore, the bands seen in FIG. 3B confirm that the light chain was properly recruited by the heavy chain and delivered to the periplasmic space. Also shown in FIG. 3 were the localization of the cytoplasmic marker protein GroEL and the periplasmic marker protein DsbC. The localization of the two marker proteins in the cytoplasm and periplasm respectively demonstrate that the subcellular fractionation was successful.

ELISA data shown in FIG. 3C clearly demonstrate that Fab activity was only detectable in DR473-A cells and not in the isogenic parent strain DHB4-A. Furthermore, a large proportion of the Fab activity was associated with the periplasmic fraction, thereby confirming TAT-specific export of the antibody fragment.

### EXAMPLE 6

### TAT-SPECIFIC EXPORT OF A VARIANT THIOREDOXIN (VTRXA)

The following example shows how the use of the TAT secretion pathway allows the transport of oxidizing equivalents (disulfide bonds) from an oxidizing cytoplasm to periplasmic proteins. The assembly of the bacterial flagella is disrupted in mutant cells impaired in the ability to form disulfide bonds in the periplasm (*i.e.* in *dsbA* or *dsbB* mutants). This is because the flagellar P-ring protein (FIgI) requires a disulfide bond in order to be assembled into the flagella structure and function properly (Dailey and Berg, 1993). This phenotype is readily apparent when cells are grown on motility plates. As shown in FIG. 4B, DR473 cells exhibit large motility 'halos' on motility plates whereas DR473-B cells lacking *dsbB* are non-motile (FIG. 4A).

TAT-dependent export of wild type thioredoxin 1 (TrxA) to the periplasm by fusion to the TorA signal sequence failed to restore motility when expressed in a strain with both an oxidizing cytoplasm and an impaired periplasmic disulfide bond formation pathway (DR473 *dsbB*) (data not shown). TAT-specific export of a more oxidizing variant of TrxA (vTrxA_{PH}) also failed to restore motility (data not shown). The more oxidizing variant of thioredoxin 1 (vTrxA_{PH}) was constructed by introducing the dipeptide sequence Ala-His within the -Cys-Gly-Pro-Cys motif in the thioredoxin active site. The substitution of Ala-His for Gly-Pro increases the redox potential of thioredoxin (Martin, 1995).

A library of TrxA with random amino acids in the protein active site (-CXXC-) was constructed as described above and fused to TorA signal sequence. A single mutant thioredoxin containing the active site sequence Cys-Ala-Cys-Cys (SEQ ID NO:7) was isolated. This trxA variant (vTrxA_{AC}) was able to partially restore motility when produced in a strain with an oxidizing cytoplasm (DR473-B in FIG. 4C as compared to the control strain DR473-B in FIG. 4A). Presumably the vTrxA_{Ac} is poised at a redox potential suitable for optimal oxidation in the cytoplasm, and for the transfer of its disulfide onto proteins once in the periplasm. Notably, when the TorA signal sequence fusion with vTrxA_{AC} was expressed in an isogenic strain with a reducing cytoplasm (MC1000 *dsbB*), motility was not restored (FIG. 4D). These results demonstrate that an oxidizing cytoplasm is necessary to deliver redox equivalents (disulfide bonds) to the periplasm via the TAT pathway. FIG. 4E confirms that the variant TrxA (vTrxA_{Ac}) was exported from the oxidizing cytoplasm into the periplasmic space. The lower band in lane 3 corresponds to the w t TrxA protein expressed from the chromosomal copy. The isogenic *trxA* mutant DR473 lacks this lower band (lanes 2 and 4).

### EXAMPLE 7

### USES OF TAT SECRETION IN COMBINATORIAL LIBRARY SCREENING

To exploit TAT secretion pathway in combinatorial library screening, an ensemble of mutated genes can be expressed with a Tat-specific leader peptide. In particular, scFv antibodies can be isolated from combinatorial libraries of antibodies either constructed from immunized animals or representative of the naive immunological repertoire. Using PCR, the heavy and light IgG chains are assembled into scFvs (Hoogenboom *et al.* 1998) and inserted into vectors suitable for periplasmic expression and cytometric screening (PECS) or for phage display, or for other combinatorial library screening format that require expression in microorganisms. After the screening is complete, clones that bind each of the two antigens and derived by screening libraries expressed with a Tat are sequenced to identify the amino acid sequences of ligand binding clones.

Thus, the present invention is directed, in one embodiment, to a method of producing at least one biologically-active heterologous polypeptide having at least one disulfide bond in a bacterial cell, comprising the steps of: constructing an expression cassette that places a leader peptide that directs protein export through the Twin Arginine Translocation pathway upstream of a gene encoding the heterologous polypeptide; and expressing the expression cassette in bacteria, wherein the heterologous polypeptide is produced in a biologically-active form. Generally, a heterologous polypeptide produced by this method will contain from about 2 to about 17 disulfide bonds. This method may be used to produce two heterologous polypeptides that are linked by at least one disulfide bond. In certain embodiments, the leader peptide is derived from a gene encoding a protein selected from the group consisting of *E. coli* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and YaeI. Alternatively, the leader peptide is derived from a gene encoding a protein selected from the group consisting of homologues of the *E. coli* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD or YaeI. In one embodiment of this method, the bacteria have an oxidizing cytoplasm. Representative bacteria which are useful in this method include *E. coli trxB* mutants, *E. coli gor* mutants, and *E. coli trxB* gor double mutants. Generally, the heterologous polypeptide is secreted from the bacterial cell, is isolatable from the periplasm of the bacterial cell or is an integral membrane protein. Optionally, the heterologous polypeptide is isolatable from the culture supernatant of the bacterial cell. Generally, the heterologous polypeptide produced by this method is a mammalian polypeptide. For example, the mammalian polypeptide may be tissue plasminogen activator or an antibody fragment. The heterologous polypeptide may be a polypeptide in native conformation, a mutated polypeptide and a truncated polypeptide.

The present invention is also directed to a method of identifying a mutated polypeptide that can reconstitute protein oxidation in a secretory compartment, comprising the steps of: generating a library of mutated polypeptides encoding a protein with oxidizing activity; constructing expression cassettes that place a leader peptide specific for the Twin Arginine Translocation pathway upstream of the mutated polypeptide; expressing the expression cassettes in bacteria that have oxidizing cytoplasm and an impaired periplasmic disulfide bond formation pathway; measuring a bacterial activity resulting from periplasmic disulfide bond formation in the bacteria; and collecting bacteria cells expressing the activity resulting from periplasmic disulfide bond formation, wherein the mutated polypeptide expressed in the collected bacteria can reconstitute protein oxidation in a secretory compartment.

The present invention is also directed to a method of screening a combinatorial library, comprising the steps of: generating a library of polypeptides of interest; constructing expression cassettes that place a leader peptide specific for the Twin Arginine Translocation pathway upstream of the polypeptides; expressing the expression cassettes in bacteria; and screening the expressed secreted polypeptides. Although a person having ordinary skill in the art could screen the expressed secreted polypeptides by any useful technique, representative techniques include periplasmic expression and cytometric screening and or phage display. Generally, the polypeptide useful in this method is a mammalian polypeptide such as an antibody fragment having from about 2 to about 17 disulfide bonds. Leader peptides useful in this technique are disclosed above.

The present invention is also directed to a method of screening a combinatorial library, comprising tne steps of: generating a library of polypeptides of interest; constructing expression cassettes comprising a leader peptide specific for the Twin Arginine Translocation pathway upstream of polypeptides; expressing the expression cassettes in bacteria that have oxidizing cytoplasm; and screening for the expression of the polypeptides in the bacteria. Generally, the polypeptide is a mammalian polypeptide. Bacteria useful in this method may have a mutation in the trxB gene. Polypeptides useful in this method may contain from about 2 to about 17 disulfide bonds.

### EXAMPLE 8

### BACTERIAL STRAINS, GROWTH AND INDUCTION CONDITIONS

Bacterial strains and plasmids that were used are described in Table 1. For monitoring the export of AP, cells grown in LB media overnight were subcultured into M9 salts supplemented with 0.2% glucose, 1 µg/ml vitamin B1, 1 mM MgSO₄, 50 µg/ml 18 amino acids (excluding methionine and cysteine) at a 100-fold dilution, and then incubated at 30°C. For the expression of scFv and Fab antibody fragments, cells were subcultured from overnight cultures into fresh LB medium (5% v/v) and then incubated at 30°C. Protein synthesis was induced by adding IPTG to a final concentration of 0.1 mM when the cells reached an OD₆₀₀~0.5. Where appropriate, the co-expression of DsbC from pBAD-ΔssDsbC was induced using 0.2% arabinose. Antibiotic selection was maintained for all markers on plasmids at the following concentrations: ampicillin, 100 µg/ml; chloramphenicol, 25 µg/ml; kanamycin, 50 µg/ml; and spectinomycin, 100 µg/ml.

**Table 1**

| **Bacterial strains and plasmids used** | | |
|---|---|---|
| ***E. coli* strain or plasmid** | **Relevant phenotype or features** | **Source** |
| DHB4 | MC1000 *phoR Δ*(*phoA*) *Pvu*II*Δ*(*malF*) 3 F'[*lacl^{q}ZYA pro*] | Laboratory stock |
| DR473 | DHB4 *ΔtrxB gor552..* Tn*10*Tet *ahpC**..Tn*10*Cm (*araC* Pₐᵣₐ*-trxB*) | Gift |
| FA113 | DHB4 *trx gor552*...Tn*10*tet^{r} *ahpC** | Gift |
| DHBA, DRA | DHB4 *dsbA::kan,* DR473 *dsbA::kan* | This study |
| DRB | DR473 *tatB::kan* | This study |
| DRC | DR473 *tatC::spec* | This study |
| pTrc99A | *trc* promoter, Co1E1 *ori,* amp^{r} | Amersham-Pharmacia |
| pAID135 | Δ2-22)AP | (Derman *et al.,* 1993) |
| pTorA-AP | ssTorA-Δ1-22)AP cloned in pTrc99A | This study |
| pKK-AP | as pTorA-AP with R11K;R12K mutation in ssTorA | This study |
| pFdnG-AP | ssFdnG-Δ1-22)-AP in pTrc99A | This study |
| pTrc99-Fab | | (Levy *et al.,* 2001) |
| pssTorA-Fab | ssTorA-V_{H}-C_{H1} and V₁-C₁ dicistronic operon in Trc99 | This study |
| pKK-Fab | as above with ssTorA(R11K;R12K) | This study |
| pBAD-ΔssdsbC | | (Levy *et al.*, 2001) |

### EXAMPLE 9

### ENZYME ACTIVITY ASSAYS

Cells expressing AP were harvested three hours after induction, treated with 100 mM iodoacetamide as above, pelleted by centrifugation and fractionated by the cold osmotic shock procedure (Sargent *et al.,* 1998). Soluble protein was quantified by the Bio-Rad protein assay, using BSA as standard. AP activity and β-galactosidase activity assays were performed as described previously (Derman *et al.,* 1993). Only data from fractionation studies in which ≥95% of the β-galactosidase activity was in the cytoplasmic fraction are reported. The trypsin resistance of AP was assessed as described previously (Sone *et al.,* 1997) except that samples were first treated with iodoacetamide. ELISA was performed according to Levy *et al.* (2001). Western blotting was performed as described previously by Chen *et al.* (2001).

### EXAMPLE 10

### ALKALINE PHOSPHATASE CAN BE EXPORTED VIA TAT ONLY IN STRAINS WITH AN OXIDIZING CYTOPLASM

In bacteria, the thioredoxin and glutaredoxin pathways maintain the cytoplasm in a highly reducing state which strongly disfavors the oxidation of protein thiols (Ritz and Beckwith, 2001). For this reason, proteins requiring disulfide bonds have to be exported into a more oxidizing environment. Alkaline phosphatase (AP) is normally secreted via a Sec-specific leader peptide into the periplasmic space where it is rapidly oxidized by DsbA to form its two disulfide bonds that are critical for the stability and catalytic activity of the protein (Sone *et al.,* 1997). Earlier studies had demonstrated that fusions of AP to Tat-specific leader peptides are not exported via the Tat pathway (Berks, 1996; Kebir and Kendall, 2002; Reinartz *et al.,* 1998; Sambasivarao et la., 1990; Stanley *et al.,* 2002). The inventors reasoned that the inability of AP fusions to be exported by the Tat pathway might be due to the fact that such proteins are normally unfolded in the cytoplasm which is maintained in a strongly reducing state that precludes the formation of disulfide bonds. To investigate this, and to examine the relationship between folding and Tat export competence, the inventors exploited the availability of mutant *E. coli* that allow the formation of disulfide bonds in the cytoplasm, or conversely disable protein oxidation in the periplasm.

Beckwith and coworkers (Bessette *et al.,* 1999) had demonstrated that disulfide bonds can form readily when AP is expressed without its signal sequence in the cytoplasm of oxidizing mutant strains such as *E.coli* DR473 (*trxB gor ahpC*). For convenience, this strain background is designated C:ox/P:ox because both the cytoplasm and the periplasm are oxidizing (FIG. 5). In a *dsbA* derivative, protein oxidation in the periplasm is impaired (Belin and Boquet, 1993). Therefore, a *dsbA* derivative of DR473 is designated C:ox/P:red. In DR473 *dsbA* cells, the formation of disulfide bonds in AP can only occur within the cytoplasm.

A set of eight leader peptides that have been shown to direct export via the Tat pathway were fused to AP and expressed in DHB4, DR473, DR473 *dsbA* and finally in DR473 *tatB::kan* and in DR473 *tatC::spec.* In the latter two strain backgrounds, the mutational inactivation of *tatB* or *tatC* is expected to impair export through the Tat pathway. Cells were grown in minimal media and the subcellular distribution of AP was determined. Following cell harvesting, cell samples were treated with 100 mM iodoacetamide to prevent the formation of disulfide bonds during fractionation by osmotic shock and the AP enzymatic activities in the cytoplasmic and periplasmic fractions were then determined. The degree of leakage of cytoplasmic components during fractionation was less than 5% as determined by the subcellular distribution of β-galactosidase activity and of GroEL (detected by Western blotting).

The fusion proteins were classified into two classes on the basis of whether the formation of active AP was strictly dependent on an oxidizing cytoplasm (Table 1). The first class of Tat leader peptides (class I; ssFdnG, ssFdoG, ssHyaA, ssTorA) was found to export AP to the periplasm in a C:ox-dependent and Tat-dependent manner. In other words, accumulation of AP activity in the periplasm only occurred in strains having both an oxidizing cytoplasm and an intact Tat apparatus. Specifically, when class I leaders were expressed in the parental strain DHB4, which has a reducing cytoplasm (*i.e.* it is C:red/P:ox) only background AP activity was observed. However, in a C:ox strain, a significant fraction of the AP activity (25-50% of total) was found in the periplasm. Importantly, the periplasmic AP activity was not dependent on DsbA and was thus virtually the same in C:ox/P:ox cells relative to C:ox/P:red cells (*i.e.,* in DR473 *dsbA*). This finding indicates that the oxidation of AP occurs only in the cytoplasm prior to export. The accumulation of active protein in the periplasm was abolished when the signature RR motif in the leader peptide was changed to a KK sequence, a substitution known to completely block export. Likewise, inactivation of either *tatB* or *tatC* in a C:ox/P:ox strain resulted in near complete loss of AP enzymatic activity in the periplasm while the cytoplasmic activity remained virtually unchanged. For one of the four class I Tat-leader peptides (ssHyaA), export was only partially blocked in a *tatB* strain.

FIG. 6A-C shows the subcellular distribution of one class I leader peptide fusion, ssFdnG-AP, in the different strain backgrounds. Inactivation of *tatC* in the C:ox strain background resulted in a lower level of cytoplasmic fusion protein. Similarly, a smaller amount of ssFdnG-AP fusion was observed in the cytoplasm of C:red cells. A higher molecular weight species corresponding to the ssFdnG-AP precursor could be resolved in 4-20% acrylamide gradient gels (FIG. 8D). This HMW species could be processed to a band with an electrophoretic mobility identical to the mature protein by incubation with trypsin, due to the presence of trypsin sensitive sites in the leader peptide (Kebir and Kendall, 2002). The mature protein was completely resistant to trypsin under conditions that result in the disappearance of full length OmpA (FIG. 6D). Further, no loss of AP enzymatic activity was detected in these studies consistent with the observation of Sone *et al.* (1997) that only the native, fully oxidized form of AP is trypsin resistant.

Four out of the eight Tat leader peptides (class II; ssDmsA, ssSufI, ssYacK and ssYcbK) exhibited high AP activity in the wt strain DHB4 (C:red/P:ox). Further analysis indicated that the class II leader peptides can engage both the Tat and the Sec pathways. Specifically the ability to engage the Sec pathway was evident by: (i) significant reduction of periplasmic AP activity in *dsbA* mutants relative to *dsbA*+ cells (compare DHB4 (C:red/P:ox) to DHB4 *dsbA* (C:red/P:red), and also DR473 (C:ox/P:ox) to DR473 *dsbA* (C:ox/P:red)). For all four class II leader peptides, the level of periplasmic AP activity in the *dsbA* mutant strains cells was approximately 60% of what was obtained in the isogenic *dsbA*+ *E. coli.* (ii) The appearance of significant periplasmic AP activity in *tatB.* or *tatC* mutants. (iii) Loss of AP activity in a *secA* conditional mutant (strain MM52 *secA51*(ts), C:red/P:ox) following upshift to the non-permissive temperature of 42°C relative to wt cells also shifted to 42°C (Tullman, DeLisa, Kawarasaki and Georgiou manuscript in preparation). The ability of class II leader peptides to also engage the Tat pathway is revealed by the presence of high AP activity in the periplasmic fraction of DR473 *dsbA* (C:ox/P:red) cells.

### EXAMPLE 11

### TAT EXPORT OF ANTIBODY FRAGMENTS

In addition to AP, class I leader peptides could mediate the export of other proteins with intra- or intermolecular disulfide bonds from the cytoplasm of C:ox strains. Single chain antibodies (scFv) contain two intermolecular disulfide bonds, one in the V_{H} and one in the V_{I} chain. The 26-10 anti-digoxin scFv (Levy *et al.,* 2001) was fused to the class I Tat leader peptide ssTorA and the accumulation of antigen binding protein in the periplasmic space of C:ox/P:ox cells was monitored by ELISA (FIG. 7). Nearly 45% of the digoxin binding activity was localized in the periplasmic space. The accumulation of active anti-digoxin scFv antibody in the periplasm was not affected in DR473 *dsbA* (C:ox/P:red)) but reduced to background levels in a *tatC* mutant.

F_{AB} antibodies are heterodimeric proteins in which the heavy chain (V_{H}-C_{H1}) and light chain (V₁-C₁) are linked by an intermolecular disulfide bond. In addition, F_{AB} proteins contain four more intrachain disulfides, two within each of the heavy and light chains. A dicistronic operon consisting of a gene encoding a ssTorA- V_{H}-C_{H1} fusion followed by the light chain (V₁-C₁) was constructed (FIG. 8A, Levy *et al.,* 2001). In this construct the light chain does not contain a leader peptide and therefore cannot be exported from the cytoplasm by itself. Induction of protein synthesis in DR473 *dsbA* (*i.e.* C:ox/P:red) was found to result in the accumulation of a small but significant amount of light chain polypeptide in the osmotic shock fraction. Western blot analysis using an anti-mouse IgG F(ab')₂ antibody specific for the F_{AB} light chain indicated that the intensity of the V₁-C₁ band in the osmotic shock fraction was approximately 15-20% of that in the cytoplasm (data not shown). Neither light chain nor F_{AB} protein could be detected in the osmotic shock fraction of a *tatC* mutant strain or from DHB4 *dsbA* (C:red/P:ox) cells.

It was reasoned that if pre-association of the heavy and light chains is required for secretion via the Tat pathway, then conditions that enhance the yield of correctly folded F_{AB} may increase the export efficiency. The yield of the anti-digoxin F_{AB} expressed in the cytoplasm of a *trxB gor aphC* mutant (the C:ox strain FA113 was used for these studies to enable co-expression of DsbC from the *ara* promoter) is increased markedly upon co-expression of a signal-sequenceless version of the periplasmic disulfide isomerase DsbC (ΔssDsbC) (Levy *et al.,* 2001). Consistent with this hypothesis, in cells co-expressing ΔssDsbC the intensity of the light chain band in the osmotic shock fraction was 70% of that in the cytoplasm (compared to 15-20% without ΔssDsbC, as noted above). A similar partitioning of the F_{AB} digoxin binding activity was detected by ELISA (FIG. 8C). In the FA113 *tatC::spec* strain, no F_{AB} could be detected in the periplasmic fraction and a significant reduction in the amount of protein remaining in the cytoplasm was also observed. The inventors and others have observed that depletion of the *tat* genes often results in inactivation or degradation of Tat substrate proteins in the cytoplasm (Angelini *et al.,* 2001; Santini *et al.,* 2001). Similarly, no light chain protein or F_{AB} binding activity could be detected in *E. coli* DHB4 (FIG. 8B and FIG. 8C) or when the RR dipeptide in ssTorA was substituted by KK.

As a final test, it was shown that functional F_{AB} protein capable of binding to the hapten digoxin is formed *in vivo.* Incubation of *E.coli* in a hypertonic solution (5X PBS) increases the outer membrane permeability such that fluorescent ligands can equilibrate within the periplasm while periplasmic proteins are unable to leak out of the cell (Chen *et al.,* 2001). Cells binding the fluorescent ligand can thus be detected by flow cytometry. C:ox/:P:ox cells expressing ssTorA-V_{H}-C_{H1} and V₁-C₁ and incubated with a digoxin-fluorescein complex exhibited a marked increase in cell fluorescence relative to control cells (C:red/P:ox, FIG. 8D). The detection of digoxin binding activity in intact cells *in situ* further rules out the possibility that formation of disulfide bonds in the F_{AB} protein may have occurred during cell fractionation.

### EXAMPLE 12

### DISCUSSION

The hallmark of the Tat pathway that sets it apart form all other modes of protein translocation across lipid bilayer membranes is the ability to export polypeptides that have already assumed a degree of stable secondary, or perhaps even tertiary, structure. The ability to genetically promote oxidative protein folding selectively in the cytoplasm or in the periplasmic space enabled the inventors to examine the requirements for export competence of proteins that: (i) do not contain cofactors and (ii) exhibit well characterized folding kinetics.

Comparison of AP accumulation in the periplasmic fraction of wt *E .coli,* mutant strains that enable the formation of disulfide bonds in the cytoplasm and their *dsbA* derivatives (Table 2) revealed that for all 8 leader peptides oxidative folding in the cytoplasm is a prerequisite for Tat export. Sone *et al* demonstrated that partially oxidized AP lacking the Cys-168-Cys-178 disulfide is proteolytically unstable and is subject to rapid degradation by DegP *in vivo* (Sone *et al.,* 1997). Thus, the stable periplasmic accumulation of the AP fusions in our studies (in a *degP*+ strain background) as well as the trypsin stability of the AP domain of ssFdnG-AP in the osmotic shock fraction (FIG. 6A-D) indicate that the exported form of the AP fusion must be fully oxidized. Based on these considerations, it was concluded that the Tat pore is capable of translocating AP that is fully oxidized and contains the native disulfide bonds. It was not possible to ascertain whether the protein is exported as a dimer or whether the association of the oxidized, folded monomers occurs following translocation into the periplasmic space, although since *in vitro,* dimerization represents a slow step in the folding of AP (Walker and Gilbert, 1994), the latter mechanism appears more plausible.

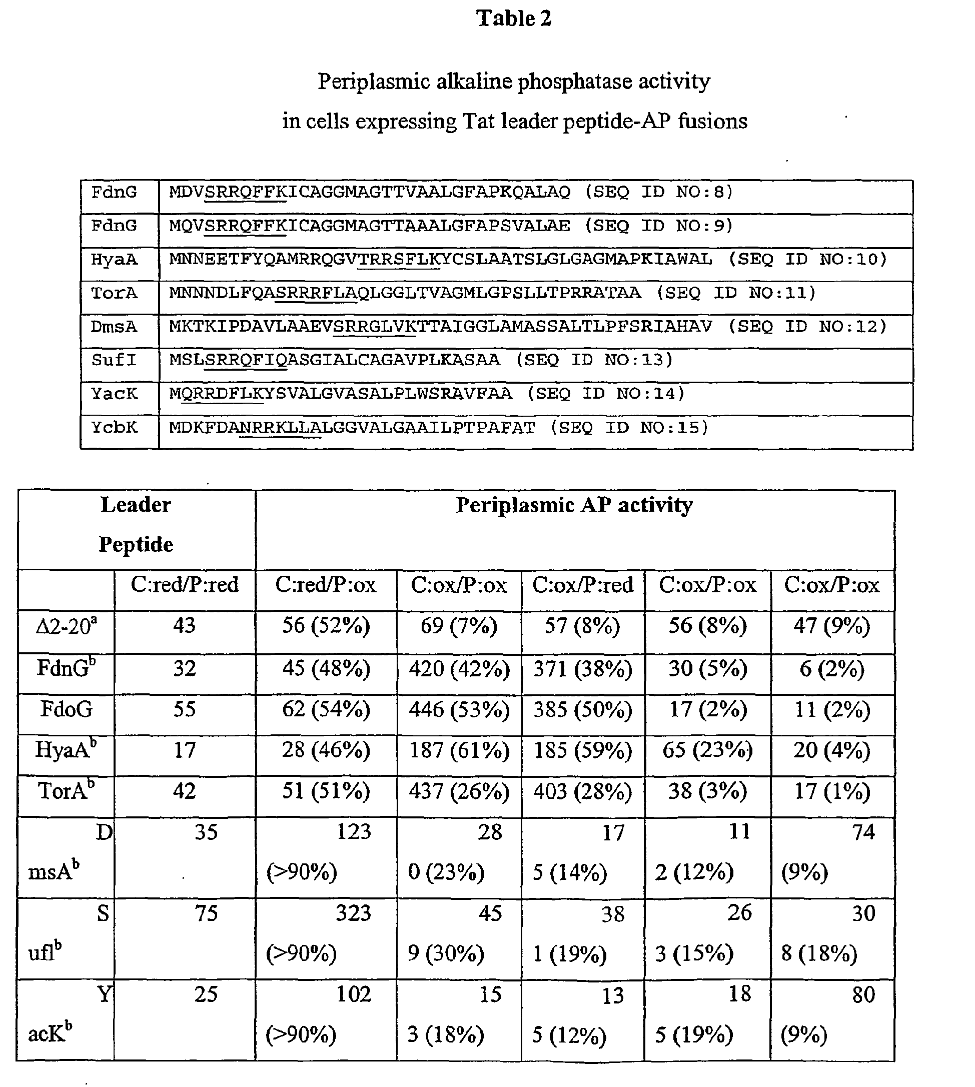

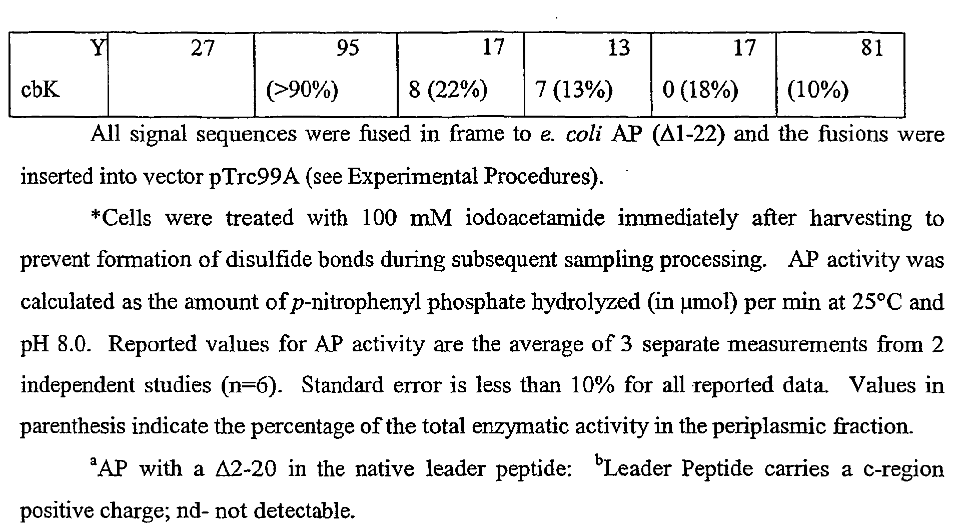

For 4/8 leader peptides (class I: ssFdnG, ssFdoG, ssHyaA and ssTorA) export occurs exclusively via the Tat pathway as evidenced by: (i) the fact that AP enzymatic activity in the osmotic shock fraction is unaffected by the presence or absence of DsbA; and (ii) export was completely abolished by the substitution of the invariant RR in the leader peptide by KK, in *tatC* mutants and, with the exception of ssHyaA, also in *tatB* mutants. The role of TatB in the export of hydrogenases has been the subject of some controversy (Walker and Gilbert, 1994). TatB is dispensable in the export of the non-physiological substrate colicin (Ize *et al.,* 2002) and also the TatB plant homologue, Hcf106, has been reported to be nonessential for the secretion of the chloroplast 16- and 23-kDa subunits of the oxygen evolving complex (Roy and barkan, 1998).

In cells expressing AP fusions to class I leader peptides approximately 26% (for ssTorA) to almost about 60% (for ssHyaA) of the total enzymatic activity in cell lysates was found in the periplasmic fraction. The observed efficiency of AP export is typical of native Tat substrates and of Tat fusions to heterologous proteins (Thomas *et al.;* 2001; Stanley *et al.,* 2002; Angelini *et al.,* 2001; DeLisa *et al.,* 2002; Yahr and Wickner, 2001). If indeed the Tat translocator exports preferentially oxidized, monomeric AP, as discussed above, then the enzymatically active protein remaining in the cytoplasm could represent dimerized protein that is incompatible with export. In addition, the accumulation of various amounts of inactive, export incompetent AP was noticed in the cytoplasm. The inactive cytoplasmic AP in C:ox cells was trypsin sensitive and its abundance was elevated at higher growth temperatures, upon expression of the fusions from high copy number plasmids and was also dependent on the leader peptide used (data not shown). In contrast, no evidence was found of inactive AP fusions in the periplasmic fraction for any of the constructs tested. In some cases (*e.g.* for ssFdnG-AP in FIG. 6D) a portion of the AP fusion protein in the periplasm migrated as a higher molecular weight band having the expected electrophoretic mobility of the precursor. This HMW species was processed by trypsin to the mature band, presumably due to the presence of sensitive sites within Tat leader peptides (Kebir and Kendall, 2002).

Class II Tat leader peptides (ssDmsA, ssSufI, ssYacK and ssYcbK) afforded some AP export from cells with a reducing cytoplasm. This AP activity was not abolished in C:red *tatB* or *tatC* mutants indicating that export of the protein to the periplasm did not involve the Tat machinery. When class II leader peptide fusions were expressed in C:ox/P:ox cells, the amount of active AP in the periplasm increased by between 42% (for ssSufI-AP) to over 100% (for ssDmsA). However, inactivation of *dsbA* in the C:ox strain DR473 resulted in a significant reduction in periplasmic AP. The two possible explanations for these results are either that translocation of misfolded or unfolded AP occurs via the Tat apparatus or that class II leader peptides can engage both the Sec and Tat translocons. The finding that the export of AP activity in C:red cells is eliminated in a *secA51*(ts) mutant strain grown at the non-permissive temperature (*E.coli* MM52 at 42°C) supports the latter hypothesis. The ability of class II leader peptides to engage both the Sec and the Tat secretion apparatus is in agreement with the recent studies by Sanders *et al.* (2001) who found that the export of heterologous cytochrome c fused to the HyaA leader peptide could proceed via the Sec pathway in the absence of the haem cofactor, whereas when the haem was enzymatically ligated in the cytoplasm, Tat became the predominant export route. Earlier studies had indicated that the presence of a positive charge at the C-terminus of Tat leader peptides can serve as a 'Sec-avoidance' signal (Cristobal *et al.,* 1999; Bogsch *et al.,* 1997). Although ssDmsA, ssSufI and ssYacK all possess a charged amino acid in this region it appears that the C-domain positive charge alone is insufficient for preventing promiscuous export via the Sec pathway, at least when these leaders are fused to AP.

Several earlier efforts to export AP via the Tat pathway had been unsuccessful (Berks, 1996; Reinartz *et al.,* 1998; Sambasivarao *et al.,* 1990; Stanley *et al.,* 2002). In some cases AP activity could be detected in the periplasmic space but export was independent of the Tat pathway (Stanley *et al.,* 2002). The analysis presented here now explains these previous observations: While the export of AP via Tat is dependent on oxidative folding in the cytoplasm, certain leader peptides are able to engage both the Sec and Tat pathways. The export flux among these two pathways presumably depends on the folding kinetics of the polypeptide, with folded molecules exported through the Tat system and proteins that have not yet reached a critical state in folding able to access the Sec pathway.

In addition to AP, the inventors found that fusions to other multidisulfide proteins, such as scFv and F_{AB} antibody fragments, become competent for export via the Tat pathway only when expressed in strains where the cytoplasm promotes oxidative protein folding. The accumulation of F_{AB} antibody in the periplasm could only be observed in C:ox strains and was dependent on an intact Tat apparatus and on the presence of a functional leader peptide on the heavy chain. The simplest explanation for these findings is that the F_{AB} first assembles in the cytoplasm where the two chains become linked by an intermolecular disulfide followed by Tat export of the folded protein. Increasing the folding efficiency of F_{AB} in the cytoplasm through the co-expression of ΔssDsbC, greatly enhanced the export efficiency of F_{AB}. The export of fully-folded Fab molecules into the periplasm even though only one of the two chains contains a leader peptide is representative of the "hitchhiker" mode of export whereby a leaderless polypeptide is exported via its association with a second polypeptide that can engage the secretion apparatus (Rodrigue *et al.,* 1999).

The results provide conclusive evidence that: (i) in the absence of folding in the cytoplasm there is no protein export via the Tat pathway; and (ii) folded proteins, of at least ~43 kDa (the size of monomeric AP), as well as proteins that must assemble subunits in the cytoplasm are exported in a folded conformation via the bacterial Tat pathway *in vivo.* The inability to export AP, scFv and F_{AB} proteins in the absence of disulfide bonds indicates the existence of a quality control mechanism that is an integral part of Tat translocation machinery. Since only the Tat proteins are required for translocation, the folding quality control mechanism must be inherent to the TatABCE proteins in the membrane. For example, a cytoplasmic domain of a Tat component may be able to function as a chaperone by binding to exposed hydrophobic regions in unfolded protein intermediates. Alternatively, interactions with aberrantly folded proteins could inhibit either the assembly of the Tat translocation pore or the gating of the pore once formed. The isolation of Tat mutations that enable the export of unfolded AP fused to class I leader peptides in Cred strains will help distinguish between these two mechanisms and is currently underway.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the compositions and methods and in the steps or in the sequence of steps of the methods described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The references listed below are incorporated herein by reference to the extent that they supplement, explain, provide a background for, or teach methodology, techniques, and/or compositions employed herein.
Angelini *et al., FEBS Lett.,* 506:8159-8164, 2001.
Belin and Boquet, *C. R. Acad. Sci. III*, 316:469-473, 1993.
Berks, *Mol. Microbiol.*, 22:393-404, 1996.
Bessette *et al., Proc. Natl., Acad. Sci. USA,* 96:13703-13708,1999.
Bogsch *et al., EMBO J.*, 16:3851-3859, 1997.
Chen *et al., Nat. Biotechnol.,* 19:537-542, 2001.
Clark and Theg, *Mol. Biol. Cell,* 8:923-934, 1997.
Cristobal *et al., EMBO J.,* 18:2982-2990,1999.
Dalbey and Robinson, *Trends Biochem. Sci.,* 24:17-22, 1999.
DeLisa *et al.*, *J. Biol. Chem.,* 277:29825-29831, 2002.
Derman *et al., Science,* 262:1744-1747, 1993.
Guzman *et al.*, J. Bacteriol., 177:4121-4130 (1995).
Halbig *et al., Eur. J. Biochem.*, 263:543-551,1999.
Halbig *et al., FEBSLett.,* 447:95-98, 1999.
Hynds *et al., J. Biol. Chem.,* 273:34868-34874, 1998.
Ize *et al., J. Mol. Biol.*, 317:327-335, 2002.
Kebir and Kendall, *Biochemistry,* 41:5573-5580, 2002.
Keegstra and Cline, *Plant Cell*, 11(4):557-570, 1999.
Levy *et al., Protein Expr. Purif.,* 23:338-347, 2001.
Mori and Cline, *J. Biol. Chem.,* 273:11405-11408, 1998.
Mossner *et al.,* J. Biol. Chem., 274:25254-25259 (1999).
Pugsley, *Microbiol. Rev.,* 57:50-108, 1993.
Reinartz *et al., Arch. Microbiol.,* 170:59-68, 1998.
Ritz and Beckwith, Annu. Rev. Microbiol., 55:21-48 (2001).
Ritz and Beckwith, *Annu. Rev. Microbiol.,* 55:5573-5580, 2002.
Robinson and Bolhuis, *Nat. Rev. Mol. Cell Biol.,* 2:350-356,2001.
Rodrigue *et al., J. Biol. Chem.,* 274:13223-13228, 1999.
Roy and Barkan, *J. Cell Biol.,* 141:385-395, 1998.
Sambasivarao *et al., J. Bacteriol.,* 172:5938-5948, 1990.
Sanders *et al., Mol. Microbiol.,* 41:241-246, 2001.
Santini *et al., J. Biol. Chem.,* 276:8159-8164, 2001.
Sargent *et al., EMBO J.,* 17:3640-3650, 1998.
Schatz and Dobberstein, Science, 271:1519-26 (1996).
Schnell and Blobel, *J*. *Cell Biol.,* 120:103-115, 1993.
Settles and Martienssen, *Trends Cell Biol.,* 8:494-501, 1998.
Settles *et al.,* Science, 278:1467-70 (1997).
Sone *et al., J. Biol. Chem.,* 272:6174-6178, 1997.
Stanley *et al., Mol, Microbiol.,* 43:1005-1021, 2002.
Stuart and Neupert, *Nature,* 406:575-577, 2000.
Thomas *et al., Mol., Microbiol.,* 39:47-53,2001.
Walker and Gilbert, *J*. *Biol. Chem.,* 269:28487-28493, 1994.
Weiner *et al., Cell,* 93:93-101, 1998.
Wexler *et al., FEBS Lett.,* 431:339-342, 1998.
Yahr and Wickner, *EMBO J.,* 20:2472-2479, 2001.

## Claims

1. A bacteria genetically transformed with an expression cassette comprising a leader peptide that directs protein export through the Twin Arginine Translocation pathway upstream of a gene encoding a heterologous polypeptide, wherein the bacteria comprises an oxidizing cytoplasm, and wherein the heterologous polypeptide is produced by the bacterial cell and comprises at least one disulfide bond.

2. The bacteria of claim 1, wherein the heterologous polypeptide contains from about 2 to about 17 disulfide bonds.

3. The bacteria of claim 1, wherein said heterologous polypeptide is produced in biologically-active form.

4. The bacteria of claim 1, wherein the leader peptide is from a gene encoding a protein selected from the group consisting of *E. coli* TorA, Sufl, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and Yael.

5. The bacteria of claim 1, wherein the leader peptide is from a gene encoding a protein selected from the group consisting of homologues of the *E. coli* TorA, SufI, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and YaeI.

6. The bacteria of claim 1, wherein the bacteria comprises a mutation that decreases or eliminates trxB gene function.

7. The bacteria of claim 1, further defined as a gram positive bacteria.

8. The bacteria of claim 1, further defined as a gram negative bacteria.

9. The bacteria of claim 1, wherein the bacteria is *E. coli.*

10. The bacteria of claim1, wherein the heterologous polypeptide is secreted from the bacteria and is isolatable from the periplasm of the bacteria or is an integral membrane protein.

11. The bacteria of claim 1, wherein the heterologous polypeptide is isolatable from a culture supernatant of said bacteria.

12. The bacteria of claim 1, wherein the heterologous polypeptide is a mammalian polypeptide.

13. The bacteria of claim 1, wherein the heterologous polypeptide is an antibody or fragment thereof.

14. The bacteria of claim 1, wherein the heterologous polypeptide is selected from the group consisting of a polypeptide in native conformation, a mutated polypeptide and a truncated polypeptide.

15. The bacteria of claim 1, wherein the heterologous polypeptide is expressed on the surface of a cytoplasmic cell membrane of said bacteria.

16. The bacteria of claim 1, wherein the heterologous polypeptide is expressed on the surface of a periplasmic cell membrane of said bacteria.

17. The bacteria of claim 1, wherein the heterologous polypeptide is expressed in the periplasm of said bacteria.

18. A method of producing at least one heterologous polypeptide comprising at least one disulfide bond in a bacterial cell, comprising the steps of :
a) constructing an expression cassette comprising a leader peptide that directs protein export through the Twin Arginine Translocation pathway upstream of a gene encoding a heterologous polypeptide; and
b) expressing said expression cassette in a bacterial cell comprising an oxidizing cytoplasm, wherein said heterologous polypeptide is produced and comprises at least one disulfide bond.

19. The method of claim 18, wherein the heterologous polypeptide contains from about 1 to about 17 disulfide bonds.

20. The method of claim 18, wherein two of said heterologous polypeptides are linked by at least one disulfide bond.

21. The method of claim 18, wherein said heterologous polypeptide is produced in biologically-active form.

22. The method of claim 18, wherein the leader peptide is from a gene encoding a protein selected from the group consisting of *E. coli* TorA, Sufl, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and Yael.

23. The method of claim 18, wherein the leader peptide is from a gene encoding a protein selected from the group consisting of homologues of the *E. coli* TorA, Suff, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and Yael.

24. The method of claim 18, wherein the heterologous polypeptide is secreted from the bacterial cell.

25. The method of claim 18, wherein the heterologous polypeptide is isolatable from the periplasm of the bacteria.

26. The method of claim 18, wherein the heterologous polypeptide is an integral membrane protein.

27. The method of claim 18, wherein the heterologous polypeptide is isolatable from the culture supernatant of said bacterial cell.

28. The method of claim 18, wherein the heterologous polypeptide is a mammalian polypeptide.

29. The method of claim 18, wherein the heterologous polypeptide is selected from the group consisting of a polypeptide in native conformation, a mutated polypeptide and a truncated polypeptide.

30. The method of claim 18, wherein the heterologous polypeptide is expressed on the surface of a cytoplasmic cell membrane of said bacteria.

31. The method of claim 18, wherein the heterologous polypeptide is expressed on the surface of a periplasmic cell membrane of said bacteria.

32. The method of claim 18, wherein the heterologous polypeptide is an antibody or fragment thereof.

33. A method of identifying a nucleic acid encoding a mutated polypeptide that can reconstitute protein oxidation in a secretory compartment, comprising the steps of :
a) obtaining expression cassettes comprising a leader peptide specific for the Twin Arginine Translocation pathway upstream of nucleic acid sequences encoding mutated polypeptides of a protein with oxidizing activity;
b) expressing said expression cassettes in bacteria that have oxidizing cytoplasm and impaired periplasmic disulfide bond formation; and
c) selecting at least a first bacteria in which said impaired periplasmic disulfide bond formation activity has been complemented by expression of said expression cassette to identify a nucleic acid sequence encoding a mutated polypeptide that can reconstitute protein oxidation in a secretory compartment.

34. A method of screening a combinatorial library, comprising the steps of :
a) generating a library of polypeptides of interest;
b) constructing expression cassettes that place a leader peptide specific for the Twin Arginine Translocation pathway upstream of a gene encoding said polypeptides ;
c) expressing said expression cassettes in bacteria comprising an oxidizing cytoplasm ; and
d) screening for expressed secreted polypeptides.

35. The method of claim 34, wherein said screening for expression is by the method of periplasmic expression and cytometric screening or phage display.

36. The method of claim 34, wherein said polypeptide is a mammalian polypeptide.

37. The method of claim 36, wherein said mammalian polypeptide is an antibody or fragment thereof.

38. The method of claim 34, wherein said leader peptide is derived from a gene encoding a protein selected from the group consisting of *E. coli,* TorA, Sufl, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and Yael.

39. The method of claim 34, wherein said leader peptide is derived from a gene encoding a protein selected from the group consisting of homologues of the *E. coli,* TorA, Sufl, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD and Yael.

## Patentansprüche

1. Bakterium, das mit einer Expressionskassette genetisch transformiert ist, deren Leitpeptid den Proteinexport Über den TAT-Weg (TAT = Twin Arginine Translocation) oberhalb eines ein heterologes Polypeptid codierenden Gens regelt, wobei das Bakterium aus einem oxidierenden Zytoplasma besteht und das heterologe Polypeptid von der Bakterienzelle erzeugt wird und mindestens eine Disulfidbindung umfasst.

2. Bakterium nach Anspruch 1, bei dem das heterologe Polypeptid etwa 2 bis etwa 17 Disulfidbindungen enthält.

3. Bakterium nach Anspruch 1, bei dem das heterologe Polypeptid in biologisch aktiver Form erzeugt wird.

4. Bakterium nach Anspruch 1, bei dem das Leitpeptid von einem Gen stammt, das ein Protein ausgewählt aus der Gruppe bestehend aus *E. coli* TorA, SufI, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD und Yael codiert.

5. Bakterium nach Anspruch 1, bei dem das Leitpeptid von einem Gen stammt, das ein Protein ausgewählt aus der Gruppe bestehend aus Homologen von *E. coli* TorA, SufI, YacK, YdhX, YdcG, WcaW, YcdB, YaeI, HyaA, HybO, HybA, NapG, NrfC, YagT, YdhX, BisZ, NapA, DmsA, YnfE, YntF, FdnG, FdoG, YahJ, AmiA, AmiC, YcdB, YedY, FhuD und YaeI codiert.

6. Bakterium nach Anspruch 1, wobei das Bakterium eine Mutation umfasst, die die Funktion des trxB-Gens abschwächt oder ausschaltet.

7. Bakterium nach Anspruch 1, weiterhin definiert als gram-positives Bakterium.

8. Bakterium nach Anspruch 1, weiterhin definiert als gram-negatives Bakterium.

9. Bakterium nach Anspruch 1, wobei das Bakterium *E. coli* ist.

10. Bakterium nach Anspruch 1, bei dem das heterologe Polypeptid von dem Bakterium sezerniert wird und vom Periplasma des Bakteriums isoliert werden kann oder ein integriertes Membranprotein ist.

11. Bakterium nach Anspruch 1, bei dem das heterologe Polypeptid aus einem Kulturüberstand des Bakteriums isoliert werden kann.

12. Bakterium nach Anspruch 1, bei dem das heterologe Polypeptid ein Säugetierpolypeptid ist.

13. Bakterium nach Anspruch 1, bei dem das heterologe Polypeptid ein Antikörper oder ein Fragment davon ist.

14. Bakterium nach Anspruch 1, bei dem das heterologe Polypeptid ausgewählt ist aus der Gruppe bestehend aus einem Polypeptid nativer Struktur, einem mutierten Polypeptid und einem trunkierten Polypeptid.

15. Bakterium nach Anspruch 1, bei dem das heterologe Polypeptid auf der Oberfläche einer zytoplasmatischen Zellmembran des Bakteriums exprimicrt wird.

16. Bakterium nach Anspruch 1, bei dem das heterologe Polypeptid auf der Oberfläche einer periplasmatischen Zellmembran des Bakteriums exprimiert wird.

17. Bakterium nach Anspruch 1, bei dem das heterologe Polypeptid im Periplasma des Bakteriums exprimiert wird.

18. Verfahren zur Herstellung mindestens eines heterologen Polypeptids mit mindestens einer Disulfidbindung in einer Bakterienzelle, das folgende Schritte umfasst:
a) Konstruktion einer Expressionskassette mit einem Leitpeptid, das den Proteinexport über den TAT-Weg oberhalb eines ein heterologes Polypeptid codierenden Gens regelt; und
b) Expression der Expressionskassette in einer Bakterienzelle mit einem oxidierenden Zytoplasma, wobei das heterologe Polypeptid erzeugt wird und mindestens eine Disulfidbindung umfasst.

19. Verfahren nach Anspruch 18, bei dem das heterologe Polypeptid etwa 1 bis etwa 17 Disulfidbindungen enthält.

20. Verfahren nach Anspruch 18, bei dem zwei der heterologen Polypeptide durch mindestens eine Disulfidbindung verbunden sind.

21. Verfahren nach Anspruch 28, bei dem das heterologe Polypeptid in biologisch aktiver Form erzeugt wird.

22. Verfahren nach Anspruch 18, bei dem das Leitpeptid von einem Gen stammt, das ein Protein ausgewählt aus der Gruppe bestehend aus *E. coli* TorA, Sufl, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD und YaeI codiert.

23. Verfahren nach Anspruch 18, bei dem das Leitpeptid von einem Gen stammt, das ein Protein ausgewählt aus der Gruppe bestehend aus Homologen von *E. coli* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD und YaeI codiert.

24. Verfahren nach Anspruch 18, bei dem das heterologe Polypeptid von der Bakterienzelle sezerniert wird.

25. Verfahren nach Anspruch 18, bei dem das heterologe Polypeptid aus dem Periplasma des Bakteriums isoliert werden kann.

26. Verfahren nach Anspruch 18, bei dem das heterologe Polypeptid ein integriertes Membranprotein ist.

27. Verfahren nach Anspruch 18, bei dem das heterologe Polypeptid aus dem Kulturüberstand der Bakterienzelle isoliert werden kann.

28. Verfahren nach Anspruch 18, bei dem das heterologe Polypeptid ein Säugetierpolypeptid ist.

29. Verfahren nach Anspruch 18, bei dem das heterologe Polypeptid ausgewählt ist aus der Gruppe bestehend aus einem Polypeptid nativer Struktur, einem mutierten Polypeptid und einem trunkierten Polypeptid.

30. Verfahren nach Anspruch 18, bei dem das heterologe Polypeptid auf der Oberfläche einer zytoplasmatischen Zellmembran des Bakteriums exprimiert wird.

31. Verfahren nach Anspruch 18, bei dem das heterologe Polypeptid auf der Oberfläche einer periplasmatischen Zellmembran des Bakteriums exprimiert wird.

32. Verfahren nach Anspruch 18, bei dem das heterologe Polypeptid ein Antikörper oder ein Fragment davon ist.

33. Verfahren zur Identifizierung einer Nukleinsäure, die ein mutiertes Polypeptid, das die Proteinoxidation in einem Sezernierungskompartment wiederherstellen kann, codiert, das folgende Schritte umfasst:
a) Gewinnung von Expressionskassetten mit einem Leitpeptid, das für den TAT-Weg oberhalb von Nukleinsäuresequenzen, die mutierte Polypeptide eines Proteins mit oxidierender Wirkung codieren, spezifisch ist;
b) Expression der Expressionskassetten in Bakterien mit oxidierendem Zytoplasma und beeinträchtigter Bildung periplasmatischer Disulfidbindungen; und
c) Auswahl mindestens eines ersten Bakteriums, bei dem die beeinträchtigte Bildungsaktivität periplasmatischer Disulfidbindungen durch Expression der Expressionskassette komplementiert wird, um eine Nukleinsäuresequenz zu identifizieren, die ein mutiertes Polypeptid codiert, das die Proteinoxidation in einem Sezernierungskompartment wiederherstellen kann.

34. Verfahren zur Durchsuchung einer kombinatorischen Bibliothek, das folgende Schritte umfasst:
a) Erzeugung einer Bibliothek in Frage kommender Polypeptide;
b) Konstruktion von Expressionskassetten mit einem Leitpeptid, das für den TAT-Weg oberhalb eines die Polypeptide codierenden Gens spezifisch ist;
c) Expression der Expressionskassetten, in Bakterien mit einem oxidierenden Zytoplasma; und
d) Durchsuchung nach exprimierten sezernierten Polypeptiden.

35. Verfahren nach Anspruch 34, bei dem die Durchsuchung nach Expression nach dem Verfahren der periplasmatischen Expression und der zytometrischen Durchsuchung oder Phagendarstellung erfolgt.

36. Verfahren nach Anspruch 34, bei dem das Polypeptid ein Säugetierpolypeptid ist.

37. Verfahren nach Anspruch 36, bei dem das Säugetierpolypeptid ein Antikörper oder ein Fragment davon ist.

38. Verfahren nach Anspruch 34, bei dem das Leitpeptid von einem Gen stammt, das ein Protein ausgewählt: aus der Gruppe bestehend aus *E*. *coli* TorA*,* SufI, YacK, YdhX, YdcG, WcaM, YcdB, YaeI, HyaA, HybO, HvbA, NapG, NrfC, YagT, YdhX, BisZ, NapA, DmsA, YnfE, YnfF, FdnG, FdoG, YahJ, AmiA, AmiC, YcdB, YedY, FhuD und YaeI codiert.

39. Verfahren nach Anspruch 34, bei dem das Leitpeptid von einem Gen stammt, das ein Protein ausgewählt aus der Gruppe bestehend aus Homologen von *E. coli* TorA, SufI, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YcdY, FhuD und YaeI codiert.

## Revendications

1. Bactérie génétiquement transformée avec une cassette d'expression comprenant un peptide de tête qui dirige l'exportation de la protéine par la voie de translocation à double arginine en amont d'un gène codant pour un polypeptide hétérologue, où la bactérie comprend un cytoplasme oxydant, et où le polypeptide hétérologue est produit par la cellule bactérienne et comprend au moins une liaison disulfure.

2. Bactérie selon la revendication 1, dans laquelle le polypeptide hétérologue contient d'environ 2 à environ 17 liaisons disulfure.

3. Bactérie selon la revendication 1, dans laquelle ledit polypeptide hétérologue est produit sous une forme biologiquement active.

4. Bactérie selon la revendication 1, dans laquelle le peptide de tête provient d'un gène codant pour une protéine choisie dans le groupe constitué par TorA, Sufl, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD et YaeI d'*E*. *coli.*

5. Bactérie selon la revendication 1, dans laquelle le peptide de tête provient d'un gène codant pour une protéine choisie dans le groupe constitué par des homologues de TorA, Sufl, YacK, YdhX, YdcG, WcaM, YcdB, Yael, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD et YaeI d'*E*. *coli.*

6. Bactérie selon la revendication 1, où la bactérie comprend une mutation qui réduit ou élimine la fonction du gène trxB.

7. Bactérie selon la revendication 1, définie en outre comme une bactérie gram-positive,

8. Bactérie selon la revendication 1, définie en outre comme une bactérie gram-négative.

9. Bactérie selon la revendication 1, la bactérie étant *E*. *coli.*

10. Bactérie selon la revendication 1, où le polypeptide hétérologue est sécrété par la bactérie et peut être isolé à partir du périplasme de la bactérie ou est une protéine membranaire intégrale.

11. Bactérie selon la revendication 1, où le polypeptide hétérologue peut être isolé à partir d'un surnageant de culture de ladite bactérie.

12. Bactérie selon la revendication 1, dans laquelle le polypeptide hétérologue est un polypeptide de mammifère.

13. Bactérie selon la revendication 1, dans laquelle le polypeptide hétérologue est un anticorps ou un fragment d'anticorps.

14. Bactérie selon la revendication 1, dans laquelle le polypeptide hétérologue est choisi dans le groupe constitué par un polypeptide dans sa conformation native, un polypeptide muté et un polypeptide tronqué.

15. Bactérie selon la revendication 1, où le polypeptide hétérologue est exprimé sur la surface d'une membrane cellulaire cytoplasmique de ladite bactérie.

16. Bactérie selon la revendication 1, où le polypeptide hétérologue est exprimé sur la surface d'une membrane cellulaire périplasmique de ladite bactérie.

17. Bactérie selon la revendication 1, où le polypeptide hétérologue est exprimé dans le périplasme de ladite bactérie.

18. Procédé de production d'au moins un polypeptide hétérologue comprenant au moins une liaison disulfure dans une cellule bactérienne, comprenant les étapes de:
a) construction d'une cassette d'expression comprenant un peptide de tête qui dirige l'exportation de la protéine par la voie de translocation à double arginine en amont d'un gène codant pour un polypeptide hétérologue; et
b) expression de ladite cassette d'expression dans une cellule bactérienne comprenant un cytoplasme oxydant, dans laquelle ledit polypeptide hétérologue est produit et comprend au moins une liaison disulfure.

19. Procédé selon la revendication 18, dans lequel le polypeptide hétérologue contient d'environ 1 à environ 17 liaisons disulfure.

20. Procédé selon la revendication 18, dans lequel deux desdits polypeptides hétérologues sont liés par au moins une liaison disulfure.

21. Procédé selon la revendication 18, dans lequel ledit polypeptide hétérologue est produit sous une forme biologiquement active.

22. Procédé selon la revendication 18, dans lequel le peptide de tête provient d'un gène codant pour une protéine choisie dans le groupe constitué par TorA, Sufl, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD et YaeJ d'*E*. *coli.*

23. Procédé selon la revendication 18, dans lequel le peptide de tête provient d'un gène codant pour une protéine choisie dans le groupe constitué par des homologues de TorA, Sufl, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD et Yael d'*E. coli.*

24. Procédé selon la revendication 18, dans lequel le polypeptide hétérologue est sécrété par la cellule bactérienne.

25. Procédé selon la revendication 18, dans lequel le polypeptide hétérologue peut être isolé à partir du périplasme de la bactérie.

26. Procédé selon la revendication 18, dans lequel le polypeptide hétérologue est une protéine membranaire intégrale.

27. Procédé selon la revendication 18, dans lequel le polypeptide hétérologue peut être isolé à partir du surnageant de culture de ladite cellule bactérienne.

28. Procédé selon la revendication 18, dans lequel le polypeptide hétérologue est un polypeptide de mammifère.

29. Procédé selon la revendication 18, dans lequel le polypeptide hétérologue est choisi dans le groupe constitué par un polypeptide dans sa conformation native, un polypeptide muté et un polypeptide tronqué.

30. Procédé selon la revendication 18, dans lequel le polypeptide hétérologue est exprimé sur la surface d'une membrane cellulaire cytoplasmique de ladite bactérie.

31. Procédé selon la revendication 18, dans lequel le polypeptide hétérologue est exprimé sur la surface d'une membrane cellulaire périplasmique de ladite bactérie.

32. Procédé selon la revendication 18, dans lequel le polypeptide hétérologue est un anticorps ou un fragment d'anticorps.

33. Procédé d'identification d'un acide nucléique codant pour un polypeptide muté qui peut reconstituer l'oxydation des protéines dans un compartiment sécrétoire, comprenant les étapes de:
a) obtention de cassettes d'expression comprenant un peptide de tête spécifique pour la voie de translocation à double arginine en amont de séquences d'acides nucléiques codant pour des polypeptides mutés d'une protéine ayant une activité oxydante;
b) expression desdites cassettes d'expression dans des bactéries qui ont un cytoplasme oxydant et une formation de liaisons disulfure périplasmique insuffisante; et
c) sélection d'au moins une première bactérie dans laquelle ladite activité de formation de liaisons disulfure périplasmique insuffisante a été complémentée par l'expression de ladite cassette d'expression pour identifier une séquence d'acide nucléique codant pour un polypeptide muté capable de reconstituer l'oxydation des protéines dans un compartiment sécrétoire.

34. Procédé de criblage d'une banque combinatoire, comprenant les étapes de:
a) production d'une banque de polypeptides d'intérêt;
b) construction de cassettes d'expression qui placent un peptide de tête spécifique de la voie de translocation à double arginine en amont d'un gène codant pour lesdits polypeptides;
c) expression desdites cassettes d'expression dans des bactéries comprenant un cytoplasme oxydant; et
d) criblage pour obtenir les polypeptides sécrétés exprimés.

35. Procédé selon la revendication 34, dans lequel ledit criblage de l'expression s'effectue par le procédé de l'expression périplasmique et du criblage cytométrique ou par présentation par les phages ("phage display").

36. Procédé selon la revendication 34, dans lequel ledit polypeptide est un polypeptide de mammifère.

37. Procédé selon la revendication 36, dans lequel ledit polypeptide de mammifère est un anticorps ou un fragment d'anticorps.

38. Procédé selon la revendication 34, dans lequel ledit peptide de tête provient d'un gène codant pour une protéine choisie dans le groupe constitué par TorA, Sufl, YacK, YdhX, YdcG, WcaM, YcdB, YacI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD et YaeI d'*E. coli.*

39. Procédé selon la revendication 34, dans lequel ledit peptide de tête provient d'un gène codant pour une protéine choisie dans le groupe constitué par des homologues de TorA, Sufl, YacK, YdhX, YdcG, WcaM, YcdB, YaeI, HyaA, HybO, HybA, NapG, NrfC, YagT, YdhX, BisZ, NapA, DmsA, YnfE, YnfF, FdnG, FdoG, YahJ, AmiA, AmiC, YcdB, YedY, FhuD et YaeI d'*E. coli.*


### SEQUENCE LISTING

<110> GEORGIOU, GEORGE
   MASIP, LUIS
   DELISA, MATTHEW
<120> SECRETION OF PROTEINS WITH MULTIPLE DISULFIDE BONDS IN BACTERIA AND USES THEREOF
<130> CLFR:018WO
<140> UNKNOWN
   <141> 2003-03-24
<150> 60/367,130
   <151> 2002-03-23
<160> 15
<170> PatentIn Ver. 2.1
<210> 1
   <211> 32
   <212> DNA
   <213> Artificial Sequence
<220>
   <223> Description of Artificial Sequence: Synthetic Primer
<400> 1
   ctctcgtcga catgagcgat aaaattattc ac 32
<210> 2
   <211> 30
   <212> DNA
   <213> Artificial Sequence
<220>
   <223> Description of Artificial Sequence: Synthetic Primer
<400> 2
   ctctcaagct tacgccaggt tagcgtcgag 30
<210> 3
   <211> 32
   <212> DNA
   <213> Artificial Sequence
<220>
   <223> Description of Artificial Sequence: Synthetic Primer
<400> 3
   ctctcgtcga catgagcgat aaaattattc ac 32
<210> 4
   <211> 20
   <212> DNA
   <213> Artificial Sequence
<220>
   <223> Description of Artificial Sequence: Synthetic Primer
<400> 4
   ctctgcccag aaatcgacga 20
<210> 5
   <211> 52
   <212> DNA
   <213> Artificial Sequence
<220>
   <223> Description of Artificial Sequence: Synthetic Primer
<220>
   <221> modified_base
   <222> (27) .. (31)
   <223> N = A, C, G or T/U
<400> 5
   tcgtcgattt ctgggcagag tggtgcnnnn nntgcaaaat gatcgccccg at 52
<210> 6
   <211> 30
   <212> DNA
   <213> Artificial Sequence
<220>
   <223> Description of Artificial Sequence: Synthetic Primer
<400> 6
   ctctcaagct tacgccaggt tagcgtcgag 30
<210> 7
   <211> 4
   <212> PRT
   <213> Artificial Sequence
<220>
   <223> Description of Artificial Sequence: Synthetic Peptide
<400> 7
   Cys Ala Cys Cys
    1
<210> 8
   <211> 34
   <212> PRT
   <213> Artificial Sequence
<220>
   <223> Description of Artificial Sequence: Synthetic Peptide
<400> 8
   Met Asp Val Ser Arg Arg Gln Phe Phe Lys Ile Cys Ala Gly Gly Met
    1               5                  10                  15
   Ala Gly Thr Thr Val Ala Ala Leu Gly Phe Ala Pro Lys Gln Ala Leu
               20                  25                  30
   Ala Gln
<210> 9
   <211> 34
   <212> PRT
   <213> Artificial Sequence
<220>
   <223> Description of Artificial Sequence: Synthetic Peptide
<400> 9
   Met Gln Val Ser Arg Arg Gln Phe Phe Lys Ile Cys Ala Gly Gly Met
   1 5 10 15
   Ala Gly Thr Thr Ala Ala Ala Leu Gly Phe Ala Pro Ser Val Ala Leu
   20 25 30
   Ala Glu
<210> 10
   <211> 46
   <212> PRT
   <213> Artificial Sequence
<220>
   <223> Description of Artificial Sequence: Synthetic Peptide
<400> 10
   Met Asn Asn Glu Glu Thr Phe Tyr Gln Ala Met Arg Arg Gln Gly Val
   1 5 10 15
   Thr Arg Arg Ser Phe Leu Lys Tyr Cys Ser Leu Ala Ala Thr Ser Leu
   20 25 30
   Gly Leu Gly Ala Gly Met Ala Pro Lys Ile Ala Trp Ala Leu
   35 40 45
<210> 11
   <211> 40
   <212> PRT
   <213> Artificial Sequence
<220>
   <223> Description of Artificial Sequence: Synthetic Peptide
<400> 11
   Met Asn Asn Asn Asp Leu Phe Gln Ala Ser Arg Arg Arg Phe Leu Ala
   1 5 10 15
   Gln Leu Gly Gly Leu Thr Val Ala Gly Met Leu Gly Pro Ser Leu Leu
   20 25 30
   Thr Pro Arg Arg Ala Thr Ala Ala
   35 40
<210> 12
   <211> 46
   <212> PRT
   <213> Artificial Sequence
<220>
   <223> Description of Artificial Sequence: Synthetic Peptide
<400> 12
   Met Lys Thr Lys Ile Pro Asp Ala Val Leu Ala Ala Glu Val Ser Arg
   1 5 10 15
   Arg Gly Leu Val Lys Thr Thr Ala Ile Gly Gly Leu Ala Met Ala Ser
   20 25 30
   Ser Ala Leu Thr Leu Pro Phe Ser Arg Ile Ala His Ala Val
   35 40 45
<210> 13
   <211> 28
   <212> PRT
   <213> Artificial Sequence
<220>
   <223> Description of Artificial Sequence: Synthetic Peptide
<400> 13
   Met Ser Leu Ser Arg Arg Gln Phe Ile Gln Ala Ser Gly Ile Ala Leu
   1 5 10 15
   Cys Ala Gly Ala Val Pro Leu Lys Ala Ser Ala Ala
   20 25
<210> 14
   <211> 29
   <212> PRT
   <213> Artificial Sequence
<220>
   <223> Description of Artificial Sequence: Synthetic Peptide
<400> 14
   Met Gln Arg Arg Asp Phe Leu Lys Tyr Ser Val Ala Leu Gly Val Ala
   1 5 10 15
   Ser Ala Leu Pro Leu Trp Ser Arg Ala Val Phe Ala Ala
   20 25
<210> 15
   <211> 31
   <212> PRT
   <213> Artificial Sequence
<220>
   <223> Description of Artificial Sequence: Synthetic Peptide
<400> 15
   Met Asp Lys Phe Asp Ala Asn Arg Arg Lys Leu Leu Ala Leu Gly Gly
   1 5 10 15
   Val Ala Leu Gly Ala Ala Ile Leu Pro Thr Pro Ala Phe Ala Thr
   20 25 30

### SEQUENCE LISTING

<110> Research Development Foundation
<120> SECRETION OF PROTEINS WITH MULTIPLE DISULFIDE BONDS IN BACTERIA AND USES THEREOF
<130> APM/JM/7709-EP
<140> EP 03716803.6
   <141> 2003-03-24
<150> PCT/US2003/008969
   <151> 2003-03-24
<150> US 60/367,130
   <151> 2002-03-23
<160> 15
<170> PatentIn Ver. 2.1
<210> 1
   <211> 32
   <212> DNA
   <213> Artificial Sequence
<220>
   <221> source
   <223> /note= "Description of Artificial Sequence: Synthetic Primer"
<400> 1
   ctctcgtcga catgagcgat aaaattattc ac 32
<210> 2
   <211> 30
   <212> DNA
   <213> Artificial Sequence
<220>
   <221> source
   <223> /note= "Description of Artificial Sequence: Synthetic Primer"
<400> 2
   ctctcaagct tacgccaggt tagcgtcgag 30
<210> 3
   <211> 32
   <212> DNA
   <213> Artificial Sequence
<220>
   <221> source
   <223> /note= "Description of Artificial Sequence: Synthetic Primer"
<400> 3
   ctctcgtcga catgagcgat aaaattattc ac 32
<210> 4
   <211> 20
   <212> DNA
   <213> Artificial Sequence
<220>
   <221> source
   <223> /note= "Description of Artificial Sequence: Synthetic Primer"
<400> 4
   ctctgcccag aaatcgacga 20
<210> 5
   <211> 52
   <212> DNA
   <213> Artificial Sequence
<220>
   <221> source
   <223> /note= "Description of Artificial Sequence: Synthetic Primer"
<220>
   <221> modified_base
   <222> (27)..(31)
   <223> N = A, C, G or T/U
<400> 5
   tcgtcgattt ctgggcagag tggtgcnnnn nntgcaaaat gatcgccccg at 52
<210> 6
   <211> 30
   <212> DNA
   <213> Artificial Sequence
<220>
   <221> source
   <223> /note= "Description of Artificial Sequence: Synthetic Primer"
<400> 6
   ctctcaagct tacgccaggt tagcgtcgag 30
<210> 7
   <211> 4
   <212> PRT
   <213> Artificial Sequence
<220>
   <221> source
   <223> /note= "Description of Artificial Sequence: Synthetic Peptide"
<400> 7
   Cys Ala Cys Cys
   1
<210> 8
   <211> 34
   <212> PRT
   <213> Artificial Sequence
<220>
   <221> source
   <223> /note= "Description of Artificial Sequence: Synthetic Peptide"
<400> 8
   Met Asp Val Ser Arg Arg Gln Phe Phe Lys Ile Cys Ala Gly Gly Met
   1 5 10 15
   Ala Gly Thr Thr Val Ala Ala Leu Gly Phe Ala Pro Lys Gln Ala Leu
   20 25 30
   Ala Gln
<210> 9
   <211> 34
   <212> PRT
   <213> Artificial Sequence
<220>
   <221> source
   <223> /note= "Description of Artificial Sequence: Synthetic Peptide"
<400> 9
   Met Gln Val Ser Arg Arg Gln Phe Phe Lys Ile Cys Ala Gly Gly Met
   1 5 10 15
   Ala Gly Thr Thr Ala Ala Ala Leu Gly Phe Ala Pro Ser Val Ala Leu
   20 25 30
   Ala Glu
<210> 10
   <211> 46
   <212> PRT
   <213> Artificial Sequence
<220>
   <221> source
   <223> /note= "Description of Artificial Sequence: Synthetic Peptide"
<400> 10
   Met Asn Asn Glu Glu Thr Phe Tyr Gln Ala Met Arg Arg Gln Gly Val
   1 5 10 15
   Thr Arg Arg Ser Phe Leu Lys Tyr Cys Ser Leu Ala Ala Thr Ser Leu
   20 25 30
   Gly Leu Gly Ala Gly Met Ala Pro Lys Ile Ala Trp Ala Leu
   35 40 45
<210> 11
   <211> 40
   <212> PRT
   <213> Artificial Sequence
<220>
   <221> source
   <223> /note= "Description of Artificial Sequence: Synthetic Peptide"
<400> 11
   Met Asn Asn Asn Asp Leu Phe Gln Ala Ser Arg Arg Arg Phe Leu Ala
   1 5 10 15
   Gln Leu Gly Gly Leu Thr Val Ala Gly Met Leu Gly Pro Ser Leu Leu
   20 25 30
   Thr Pro Arg Arg Ala Thr Ala Ala
   35 40
<210> 12
   <211> 46
   <212> PRT
   <213> Artificial Sequence
<220>
   <221> source
   <223> /note= "Description of Artificial Sequence: Synthetic Peptide"
<400> 12
   Met Lys Thr Lys Ile Pro Asp Ala Val Leu Ala Ala Glu Val Ser Arg
   1 5 10 15
   Arg Gly Leu Val Lys Thr Thr Ala Ile Gly Gly Leu Ala Met Ala Ser
   20 25 30
   Ser Ala Leu Thr Leu Pro Phe Ser Arg Ile Ala His Ala Val
   35 40 45
<210> 13
   <211> 28
   <212> PRT
   <213> Artificial Sequence
<220>
   <221> source
   <223> /note= "Description of Artificial Sequence: Synthetic Peptide"
<400> 13
   Met Ser Leu Ser Arg Arg Gln Phe Ile Gln Ala Ser Gly Ile Ala Leu
   1 5 10 15
   Cys Ala Gly Ala Val Pro Leu Lys Ala Ser Ala Ala
   20 25
<210> 14
   <211> 29
   <212> PRT
   <213> Artificial Sequence
<220>
   <221> source
   <223> /note= "Description of Artificial Sequence: Synthetic Peptide"
<400> 14
   Met Gln Arg Arg Asp Phe Leu Lys Tyr Ser Val Ala Leu Gly Val Ala
   1 5 10 15
   Ser Ala Leu Pro Leu Trp Ser Arg Ala Val Phe Ala Ala
   20 25
<210> 15
   <211> 31
   <212> PRT
   <213> Artificial Sequence
<220>
   <221> source
   <223> /note= "Description of Artificial Sequence: Synthetic Peptide"
<400> 15
   Met Asp Lys Phe Asp Ala Asn Arg Arg Lys Leu Leu Ala Leu Gly Gly
   1 5 10 15
   Val Ala Leu Gly Ala Ala Ile Leu Pro Thr Pro Ala Phe Ala Thr
   20 25 30